# EUROPEAN PATENT APPLICATION

(11) **EP 3 964 508 A1**
(43) Date of publication of application: **09.03.2022**
(21) Application number: 20794158.4
(22) Date of filing: 09.04.2020
(51) Int. Cl.: C07D 413/14, C07D 413/04, C07D 401/14, A61K 31/506, A61P 35/00, A61P 35/02

(54) **DIMERIC OR POLYMERIC FORM OF MUTANT IDH INHIBITOR**

(30) Priority: 23.04.2019 CN 201910330135
(71) Applicant: Epitas Biosciences (Shanghai) Co., Ltd., Shanghai 201203 (CN)
(72) Inventor: ZENG, Mi, Shanghai 201203 (CN); WU, Haiping, Shanghai 201203 (CN); ZHU, Weixing, Shanghai 201203 (CN)
(74) Representative: Nederlandsch Octrooibureau
(86) International application number: PCT/CN2020/084041
(87) International publication number: WO 2020/216071

(57) **Abstract**

The present invention provides a mutant IDH inhibitor in dimeric or multimeric form. Specifically, the present invention provides a compound of formula I,

Da-Wa-L-Wb-Db (I)

or a pharmaceutically acceptable salt thereof. The compound of the present invention has excellent inhibitory activity against mutant IDH1.

## Description

### Technical Field

The invention belongs to field of medicinal chemistry, and specifically relates to a mutant IDH inhibitor.

### Background

As a key rate-limiting enzyme in tricarboxylic acid cycle involved in cell energy metabolism, isocitrate dehydrogenase (IDH) catalyzes oxidative decarboxylation of isocitric acid to produce α-ketoglutaric acid (a-KG) and CO₂. There are two different subtypes of IDH, one using NAD(+) as an electron acceptor and the other using NADP (+) as the electron acceptor. Five isozymes of IDH have been reported, three of which are NAD(+)-dependent isocitrate dehydrogenases, locating in the mitochondrial matrix; and the other two of which are NADP(+)-dependent isocitrate dehydrogenases, wherein the isocitrate dehydrogenase 1 (IDH1) locates in the cytoplasm and the isocitrate dehydrogenase 2 (IDH2) locates in the mitochondria.

Many cancers have isocitrate dehydrogenase mutations, such as glioma, glioblastoma, paragangliomas, acute leukemia, prostate cancer, thyroid cancer, colon cancer, chondrosarcoma, bile duct epithelial carcinoma, peripheral T cell leukemia, melanoma, etc. And IDH1 mutations have been found in a variety of tumors. IDH1 Mutation (IDH1m) may result in the loss of normal function of IDH1 and convert α-KG into the carcinogenic metabolite 2-hydroxyglutarate (2-HG) and allow 2-HG to accumulate in mutated tumor cells. Researches have shown that the structure of α-KG is similar to that of 2-HG, and 2-HG competes with α-KG, thereby reducing the activity of α-KG-dependent enzymes, and resulting in a hypermethylation of nucleosomes and/or DNA in some key regions of the genome. Such epigenetic change is considered to interfere with a normal cell differentiation, and leads to an excessive proliferation of immature cells, thereby causing cancers. In 2009, Bleeker *et al.* have detected IDH1 mutations in 672 tumor samples obtained from different sources and 84 cell lines from different tumor lineages, and found that these mutations specifically and centrally occurred in gliomas. However, the later literature reports have shown that IDH1 mutations also exist in acute myeloid leukemia, prostate cancer, and paraganglioma and the like. Bleeker *et al.* found that in IDH1 mutation cases, R132H accounts for 86.9%, and other types such as R132C, R132G, R132L, R132V and R132S account for a small proportion.

In tumor cells containing IDH1 mutations, mutant IDH1 inhibitors may specifically bind to the catalytic domain of the mutant enzyme protein, and effectively inhibit the activity of the mutant protease and reduce the carcinogenic metabolite 2-HG in the body through allosteric inhibition, thereby inducing the demethylation of histones and/or DNA to promote tumor cell differentiation and inhibit tumor development. At present, the only marketed drug Ivosidenib (AG-120) that inhibits the activity of mutant IDH1 is a new drug developed by Agios to treat relapsed or refractory acute myeloid leukemia in adults carrying IDH1 gene mutations. The medication guide of Ivosidenib states 500 mg per day. The dosage is large, and the clinical trials conducted with the dosage on patients with relapsed or progressing IDH1 mutant gliomas did not achieve the desired effect. Patients with IDH1 mutants are basically heterozygous mutations. Therefore, there is a continuous urgent need for new, high-efficiency and low-toxicity mutant IDH1 inhibitors.

In summary, there is an urgent need in the art to develop a new, high-efficiency and low-toxicity mutant IDH1 inhibitor.

### Summary

A purpose of the present invention is to provide a new, high-efficiency and low-toxicity mutant IDH1 inhibitor.

In a first aspect, the present invention provides a compound of formula I,

Dₐ-Wₐ-L-W_{b}-D_{b} (I)

or a pharmaceutically acceptable salt thereof, wherein,
Wa and Wb are each independently absent or selected from the group consisting of O, S, NRa, CO, COO, SO, SO₂, CO-NRₐ, NRₐ-CO, SO-N(Rₐ), N(Ra)-SO, NRₐ-COO, COO-NRₐ, NRₐ-SO₂, SO₂-NRₐ, CS-NRₐ, NRₐ-CS or N(Rₐ)-CO-NRₐ;
wherein Rₐ is each independently selected from the group consisting of H, deuterium, CN, halogen, C1-C6 alkyl, C1-C6 halogenated alkyl, or substituted or unsubstituted C3-C6 cycloalkyl, preferably the substitution is C1-C6 alkyl substitution and/or halogen substitution; preferably, Rₐ is each independently selected from the group consisting of H, deuterium, -CH₃, -C₂H₅, -CH(CH₃)₂, -CH₂CH₂CH₃, cyclopropyl;
L is a linking group shown in formula II

   -(X)ₙ- (II)

   wherein,
n is an integer of 1-50, preferably an integer of 3-40;
each X is the same or different, and each X is independently selected from the group consisting of O, S, substituted or unsubstituted C1-C6 alkylene, substituted or unsubstituted C2-C6 alkenylene, substituted or unsubstituted C2-C6 alkynylene, CO, SO₂, NR_{b}, C(R_{c})₂, substituted or unsubstituted 4- to 10-membered carbocyclic ring (preferably 4- to 7-membered carbocyclic ring), substituted or unsubstituted 4- to 10-membered heterocyclic ring (preferably 4- to 7-membered heterocyclic ring), substituted or unsubstituted 6- to 12-membered aromatic ring (preferably 6-membered aromatic ring), substituted or unsubstituted 5- to 12-membered heteroaromatic ring (preferably a 5- to 7-membered heteroaromatic ring); or -Wc(T)ₖ-, wherein Wc is a trivalent group, a tetravalent group, or a pentavalent group (preferably N, CH, C, dendritic group), and k is 1, 2 or 3; T is -R_{d}-Wa-L'-Wb-D_{c}, wherein Wa and Wb are as described above;
each R_{d} is independently absent or a divalent group selected from the group consisting of substituted or unsubstituted C1-C6 alkylene, substituted or unsubstituted C2-C6 alkenylene, substituted or unsubstituted C1-C6 halogenated alkylene, substituted or unsubstituted C3-C6 cycloalkyl;
L' is absent or a linking group shown in formula II-A:

   -(Y)ₘ- (II-A)

   wherein,
m is an integer of 1-50, preferably an integer of 3-40;
each Y is the same or different, and each Y is independently selected from the group consisting of O, S, substituted or unsubstituted C1-C6 alkylene, substituted or unsubstituted C2-C6 alkenylene, substituted or unsubstituted C2-C6 alkynylene, CO, SO₂, NR_{b}, C(R_{c})₂, substituted or unsubstituted 4- to 10-membered carbocyclic ring (preferably 4- to 7-membered carbocyclic ring), substituted or unsubstituted 4- to 10-membered heterocyclic ring (preferably 4- to 7-membered heterocyclic ring), substituted or unsubstituted 6- to 12-membered aromatic ring (preferably 6-membered aromatic ring), substituted or unsubstituted 5- to 12-membered heteroaromatic ring (preferably a 5- to 7-membered heteroaromatic ring);
each R_{b} is independently selected from the group consisting of H, deuterium, C1-C6 alkyl, C1-C6 halogenated alkyl, C1-C6 alkoxy;
each R_{c} is independently selected from the group consisting of H, deuterium, halogen, C1-C6 alkyl, C1-C6 halogenated alkyl, C1-C6 alkoxy, OH, CN; (preferably, H, deuterium, C1-C4 alkyl or halogen; more preferably, H, deuterium, methyl, ethyl, propyl, isopropyl);
wherein, the heterocyclic ring or the heteroaromatic ring contains 1, 2 or 3 heteroatoms selected from O, S or N;
with the proviso that, when X is a substituted carbocyclic ring, substituted heterocyclic ring, substituted aromatic ring, substituted heteroaryl, the substituents on the carbocyclic ring, heterocyclic ring, aromatic ring or heteroaryl may optionally contain 1, 2 or 3 T, wherein T is as defined above;
unless otherwise specified, the term "substituted" means that 1 to 5 (preferably, 1 to 3) hydrogens in the group are each independently replaced by a substituent selected from the group consisting of deuterium, halogen, C1-C6 alkyl, C1-C6 halogenated alkyl, C1-C6 alkoxy, substituted or unsubstituted phenyl, substituted or unsubstituted benzyl,-N(R_{b})₂, -C(R_{c})₃, -CN, -OH, -COOR_{f}, -SO₂R_{f}, -NHC(O) R_{f}; wherein R_{f} is each independently selected from the group consisting of hydrogen, deuterium, C1-C6 alkyl, R_{b} and R_{c} are as defined above;
Dₐ, D_{b} and D_{c} are each independently an active group that inhibits mutant IDH protein.

In another preferred embodiment, Dₐ, D_{b} and D_{c} are each independently an active group that specifically inhibits mutant IDH1 protein.

In another preferred embodiment, the mutant IDH protein is a mutant IDH1 protein.

In another preferred embodiment, the "specific inhibition" means that there is no or substantially no inhibitory effect on IDH2 (such as mutant IDH2).

In another preferred embodiment, the mutant IDH1 is selected from the group consisting of IDH1/R132H and IDH1/R132C.

In another preferred embodiment, Dₐ, D_{b} and D_{c} are all the same.

In another preferred embodiment, Dₐ and D_{b} are the same.

In another preferred embodiment, Dₐ and D_{c} are the same.

In another preferred embodiment, D_{b} and D_{c} are the same.

In another preferred embodiment, each D_{c} is the same.

In another preferred embodiment, the number of the active groups in formula I is 2, 3, 4 or 5 (that is, the number of D_{c} is 0, 1, 2, or 3).

In another preferred embodiment, the active group is a monovalent or divalent group derived from an active compound.

In another preferred embodiment, Dₐ, D_{b} and each D_{c} are monovalent groups.

In another preferred embodiment, the active compound is selected from the compounds that inhibit mutant IDH protein disclosed in WO2013046136A1 (i.e., the compounds of formula (I) disclosed in WO2013046136A1).

In another preferred embodiment, the active compound is 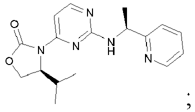 or the active compound is selected from the compounds 1-169, 170-212, 213-312, 313-442, 443a and 443b, 444-455, 456a and 456b, 457a and 457b, 458a and 458b, 459-485, 486-507, 508, 509-513, 514, 515, 516-546, 547-550, 552-559, 560-562, 563, 564-565, 566-579, 580-582, 583-586, 587-589, 590-604 disclosed in WO2013046136A1.

In another preferred embodiment, Dₐ, D_{b} and D_{c} are each independently a group shown in formula III: 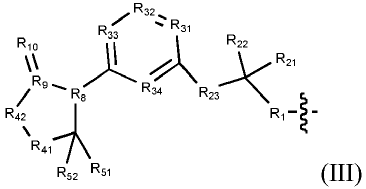 wherein,
R₁ is a divalent linking group;
R₂₁ and R₂₂ are each independently selected from the group consisting of hydrogen, deuterium, halogen, amino, cyano, substituted or unsubstituted C1-C6 alkyl, or R₂₁ and R₂₂ taken together with the carbon atom to which they are attached form a substituted or unsubstituted C3-C5 cycloalkyl; wherein the term "substituted" means that one or more (preferably, 1 to 3) H in the group are replaced by a substituent selected from the group consisting of deuterium, halogen, C1-C6 alkyl;
R₂₃ is selected from the group consisting of NR₆₂, preferably, R₂₃ is NH;
R₃₁, R₃₂, R₃₃ and R₃₄ are each independently selected from the group consisting of N, CR₆₁, preferably, CH;
R₈ is selected from the group consisting of N, CR_{61,} preferably, R₈ is N;
R₉ is C;
R₁₀ is selected from the group consisting of O, S, preferably, R₁₀ is O;
R₄₁ and R₄₂ are each independently selected from the group consisting of O, S, C(R₅₃)₂;
R₅₁ is each independently selected from the group consisting of H, deuterium, substituted or unsubstituted C1-C6 alkyl, substituted or unsubstituted phenyl, substituted or unsubstituted benzyl, substituted or unsubstituted 5- to 7-membered heteroaryl, substituted or unsubstituted diphenylmethyl (i.e., -CH(C₆H₅)₂); R₅₂ is selected from the group consisting of H, deuterium, C1-C6 alkyl;
or R₅₁ and R₅₂ taken together with the carbon atom to which they are attached form a substituted or unsubstituted C1-C6 (preferably, C1-C4) cycloalkyl, substituted or unsubstituted 5- to 7-membered heterocyclyl;
R₅₃ is each independently selected from the group consisting of H, deuterium, substituted or unsubstituted C1-C6 alkyl, phenyl, benzyl; or two R₅₃ taken together with the carbon atom to which they are attached form a substituted or unsubstituted 3- to 7-membered cycloalkyl or substituted or unsubstituted 4- to 7-membered heterocyclic ring; in the R₅₃ group, the term "substituted" means that 1 to 3 hydrogens in the group are each independently replaced by a substituent selected from the group consisting of deuterium, halogen, hydroxyl, CI-6 alkyl, C1-C6 halogenated alkyl, amino;
R₆₁ is each independently selected from the group consisting of hydrogen, deuterium, halogen (preferably, F, Cl, Br), C1-C6 alkyl, C1-C6 halogenated alkyl; and
R₆₂ is each independently selected from the group consisting of hydrogen, deuterium, C1-C6 alkyl, C1-C6 halogenated alkyl.

In another preferred embodiment, R₂₁ and R₂₂ are each independently selected from the group consisting of hydrogen, deuterium, C1-C6 alkyl, C1-C6 halogenated alkyl (preferably, one of R₂₁ and R₂₂ is hydrogen or deuterium; and the other is C1-C6 alkyl); or R₂₁ and R₂₂ taken together with the carbon atom to which they are attached form a C3-C5 cycloalkyl.

In another preferred embodiment, R₃₁ is selected from the group consisting of CH, N.

In another preferred embodiment, R₃₂ is selected from the group consisting of CH, N.

In another preferred embodiment, R₃₃ is CR₆₁; preferably, R₃₃ is CH or CF.

In another preferred embodiment, R₃₄ is N.

In another preferred embodiment, R₄₁ is C(R₅₃)₂; preferably, R₄₁ is CH₂.

In another preferred embodiment, R₄₂ is selected from the group consisting of O, S; preferably, R₄₂ is O.

In another preferred embodiment, R₅₁ is selected from the group consisting of C1-C6 alkyl, C1-C6 halogenated alkyl.

In another preferred embodiment, R₅₂ is selected from the group consisting of hydrogen, deuterium.

In another preferred embodiment, R₁ is -Ar₁-L₁-Ar₂-L₂-Ar₃-, wherein,
Ar₁, Ar₂ and Ar₃ are each independently absent or selected from the group consisting of substituted or unsubstituted C6-C20 aromatic ring, substituted or unsubstituted C3-C18 aromatic heterocyclic ring;
L₁ and L₂ are each independently absent, C1-C3 alkylene or C3-C6 cycloalkyl;
and at least one of Ar₁, Ar2 and Ar₃ is not absent;
in the R₁ group, the term "substituted" means that one or more (preferably, 1-3) H in the ring are replaced by a substituent selected from the group consisting of halogen (preferably, F, Cl, Br), C1-C6 alkyl, C1-C6 halogenated alkyl, C1-C6 alkyloxy.

In another preferred embodiment, R₁ is substituted or unsubstituted C6-C20 aryl (preferably, substituted or unsubstituted phenyl or naphthyl), substituted or unsubstituted C3-C18 heteroaryl (preferably, substituted or unsubstituted pyridyl), substituted or unsubstituted C6-C20 aryl-Li-substituted or unsubstituted C6-C20 aryl (preferably, substituted or unsubstituted biphenyl), substituted or unsubstituted C6-C20 aryl-Li-substituted or unsubstituted C3-C18 heteroaryl, or substituted or unsubstituted C3-C18 heteroaryl-Li-substituted or unsubstituted C3-C18 heteroaryl.

In another preferred embodiment, R₁ is pyridyl.

In another preferred embodiment, R₁ is a group shown in formula VI-A or formula VI-B: 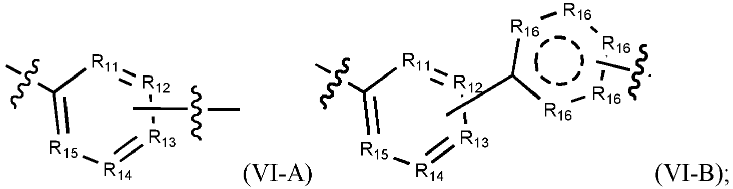
wherein, R₁₁, R₁₂, R₁₃, R₁₄ and R₁₅ are each independently selected from the group consisting of N, CR₆₁; R₁₆ is each independently selected from the group consisting of N, CR₆₃;
wherein, R₆₃ is selected from the group consisting of deuterium, halogen (preferably, F, Cl), C1-C6 alkyl, C1-C6 alkoxy, C1-C6 halogenated alkyl (preferably, fluoroalkyl).

In another preferred embodiment, R₁₁ is selected from the group consisting of N, CR₆₁ (preferably, CH); and R₁₂, R₁₃, R₁₄ and R₁₅ are CR₆₁ (preferably, CH).

In another preferred embodiment, R₁₁, R₁₂, R₁₃, R₁₄ and R₁₅ are each independently selected from the group consisting of N, CH, CF, CCl.

In another preferred embodiment, R₁₁ is N, and R₁₂, R₁₃, R₁₄ and R₁₅ are each independently selected from the group consisting of N, CH, CF, CCl.

In another preferred embodiment, R₁ is a substituted or unsubstituted divalent group selected from the group consisting of

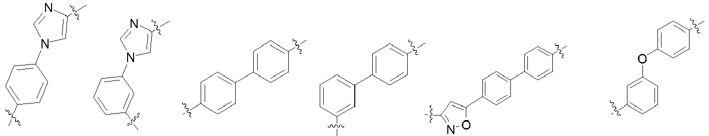

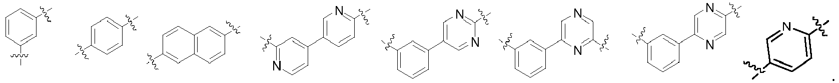

In another preferred embodiment, Dₐ, D_{b} and D_{c} are each independently a group shown in formula III-A or III-B: 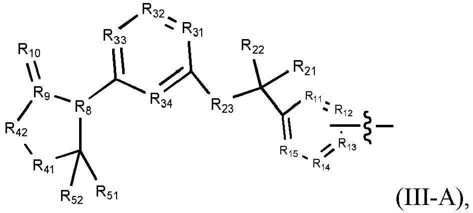 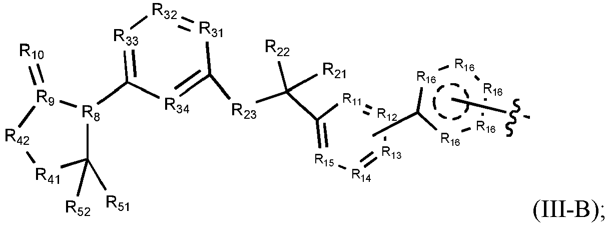 wherein, R₂₁, R₂₂, R₂₃, R₃₁, R₃₂, R₃₃, R₃₄, R₈, R₉, R₁₀, R₄₁, R₄₂, R₅₁, R₅₂, R₁₁, R₁₂, R₁₃, R₁₄, R₁₅ and R₁₆ are as defined above.

In another preferred embodiment, Dₐ, D_{b} and D_{c} are each independently a group shown in formula III-C: 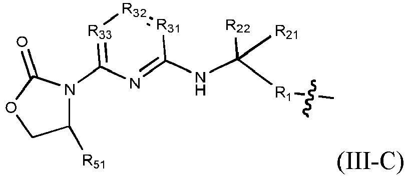 wherein, R₁, R₂₁, R₂₂, R₃₁, R₃₂, R₃₃ and R₅₁ are as defined above.

In another preferred embodiment, Dₐ, D_{b} and D_{c} are each independently a group shown in formula III-D or III-E: 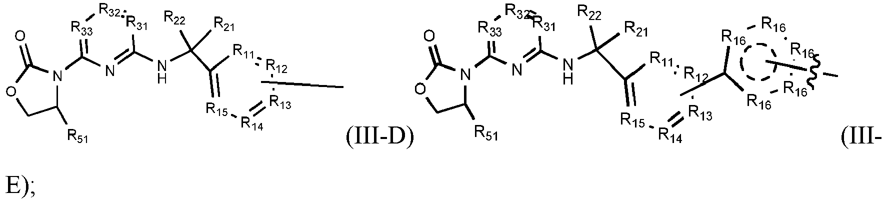 wherein, R₂₁, R₂₂, R₅₁, R₃₁, R₃₂, R₃₃, R₁₁, R₁₂, R₁₃, R₁₄, R₁₅ and R₁₆ are as defined above.

In another preferred embodiment, Dₐ, D_{b} and each D_{c} are each independently a monovalent group selected from the group consisting of

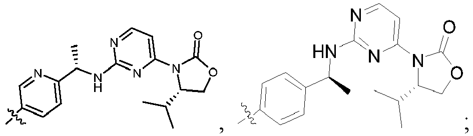

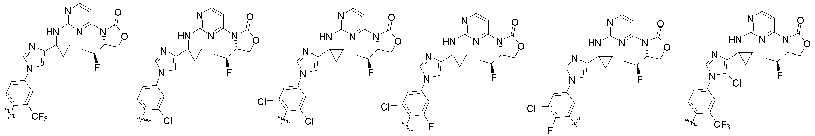

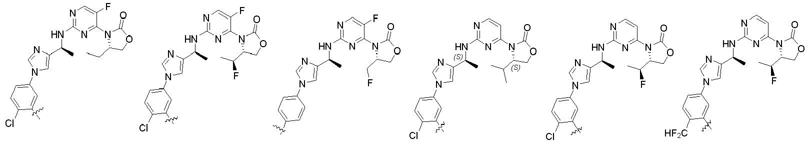

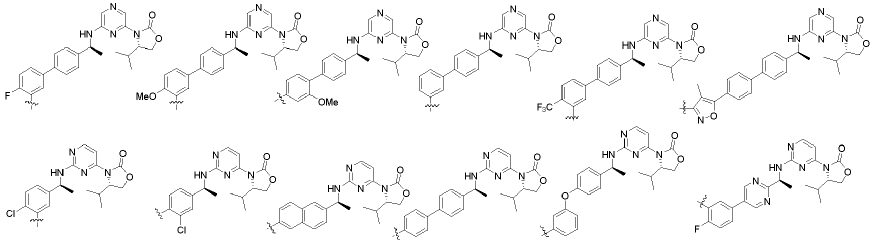

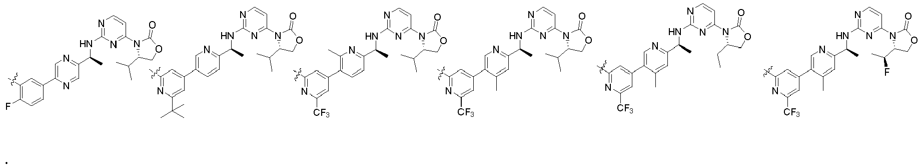

In another preferred embodiment, Dₐ, D_{b} and D_{c} are the same monovalent group. In another preferred embodiment, Dₐ, D_{b} and D_{c} are

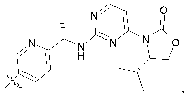

In another preferred embodiment, Dₐ and D_{b} are the same monovalent group.In another preferred embodiment, Dₐ and D_{b} are

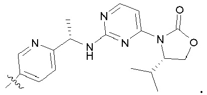

In another preferred embodiment, the substituted phenyl has 1-5 substituents selected from group (a):

(a) halogen, C1-C6 alkyl, C1-C6 halogenated alkyl, C1-C6 alkoxy, C1-C6 amino, C2-C6 alkenyl, C2-C6 alkynyl, C3-C8 cycloalkyl, OH, CN.

In another preferred embodiment, the substituted benzyl has 1-5 substituents selected from group (a).

In another preferred embodiment, each X is a divalent group.

In another preferred embodiment, the definition of X is the same as that of Y.

In another preferred embodiment, each X is selected from the group consisting of O, S, C1-C6 alkylene, NR_{b}, C(R_{c})₂, a divalent group formed by a substituted or unsubstituted 4- to 7-membered carbocyclic or heterocyclic losing two hydrogens at any position.

In another preferred embodiment, each X is selected from the group consisting of O, S, NH, C1-C6 alkylene (preferably CH₂, CH(CH₃), (CH₂)₂, CH₂CH(CH₃), (CH₂)₃).

In another preferred embodiment, each X is selected from the group consisting of O, NH or C1-C6 alkylene.

In another preferred embodiment, each X is selected from the group consisting of O or CH₂.

In another preferred embodiment, L is a linking group shown in formula II-C:

-(Y₁-Y₂-Y₃)_{S}- (II-C)

s is an integer of 1-15, preferably an integer of 3-40; more preferably an integer of 3-9;
Y₁, Y₂ and Y₃ are each independently selected from the group consisting of O, S, C1-C6 alkylene, C2-C6 alkenylene, C2-C6 alkynylene, CO, SO₂, NR_{b}, or C(R_{b})₂, or a divalent group formed by substituted or unsubstituted 4- to 10-membered carbocyclic ring (preferably 4- to 7-membered carbocyclic ring), 4- to 10-membered heterocyclic ring (preferably 4- to 7-membered heterocyclic ring), 6- to 12-membered aromatic ring (preferably 6-membered aromatic ring), or 5- to 12-membered aromatic ring (preferably a 5- to 7-membered aromatic ring) losing two hydrogens at any position including hydrogens on the same atom or hydrogens on different atoms.

In another preferred embodiment, s is an integer of 2-10, preferably an integer of 3-7.

In another preferred embodiment, Y₁, Y₂ and Y₃ are each independently selected from the group consisting of O, S, C1-C6 alkylene, NR_{b}, or C(R_{c})₂, or a divalent group formed by a substituted or unsubstituted 4- to 7-membered carbocyclic or heterocyclic losing two hydrogens at any position; and preferably are each independently absent or selected from the group consisting of O or C1-C6 alkylene.

In another preferred embodiment, Y₁, Y₂ and Y₃ are each independently selected from the group consisting of O or CH₂, and when they are adjacent groups, they are not O at the same time.

In another preferred embodiment, L is -(CH₂-CH₂-O)ₛ-, wherein s is an integer of 2-10, more preferably s is 3, 4, 5, 6 or 7.

In another preferred embodiment, at least one X is a trivalent group, a tetravalent group, or a pentavalent group.

In another preferred embodiment, at least one X is a trivalent group.

In another preferred embodiment, L is a group shown in formula II-D:

-(Y)ₚ-Z-(Y)_{q} - (II-D)

wherein,
Z is -Wc(T)ₖ-;
p is an integer of 0-50, q is an integer of 0-50, and 1≤p+q<50;
Y, Wc, T and k are as defined above.

In another preferred embodiment, p is an integer of 1-30, q is an integer of 1-30, and 2≤p+q<50.

In another preferred embodiment, p is an integer of 3-30, q is an integer of 3-30, and 6≤p+q<30.

In another preferred embodiment, the main chain (i.e., L) has a chain length of 3-50 chain atoms excluding H atoms; preferably 6-40 chain atoms; more preferably 9-30 chain atoms; most preferably 9-21 chain atoms.

In another preferred embodiment, Wa and Wb are each independently absent or selected from the group consisting of O, S, CO, COO, NH, CO-NH or NH-CO.

In another preferred embodiment, Wa and Wb are each independently absent or O.

In another preferred embodiment, the compound is a compound shown in formula IV: 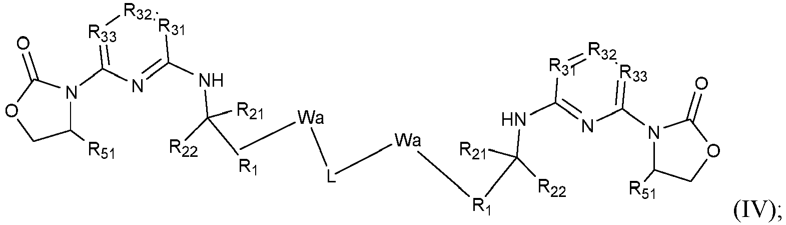 wherein,
R₁, R₂₁, R₂₂, R₃₁, R₃₂, R₃₃, R₅₁, Wa, Wb and L are as defined above.

In another preferred embodiment, the compound is shown in formula V: 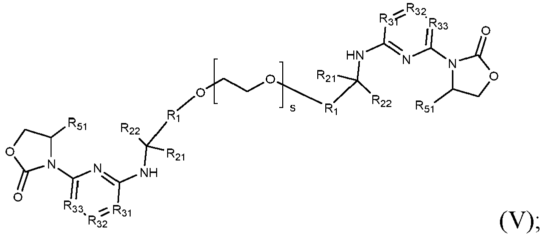 wherein, s is 3, 4, 5, 6 or 7;
R₁, R₂₁, R₂₂, R₃₁, R₃₂ and R₅₁ are as defined above.

In another preferred embodiment, the compound is a compound selected from the group consisting of

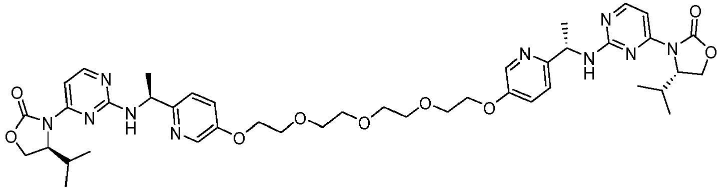

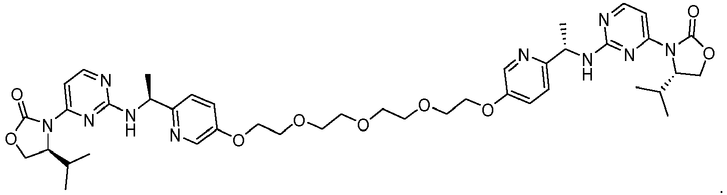

In a second aspect, the present invention provides a pharmaceutical composition, wherein the composition comprises (i) the compound as described in the first aspect, and (ii) a pharmaceutically acceptable carrier.

In a third aspect, the present invention provides a method for preparing the compound described in the first aspect, wherein the method comprises:

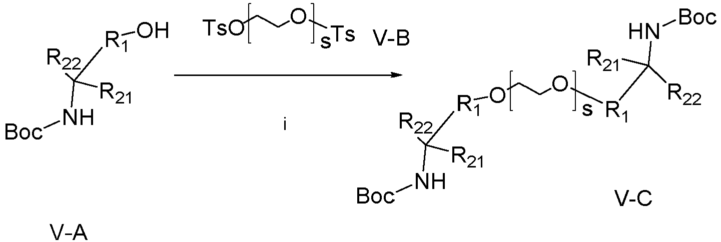

(i) reacting a compound of formula V-A with formula V-B to obtain a compound of formula V-C;

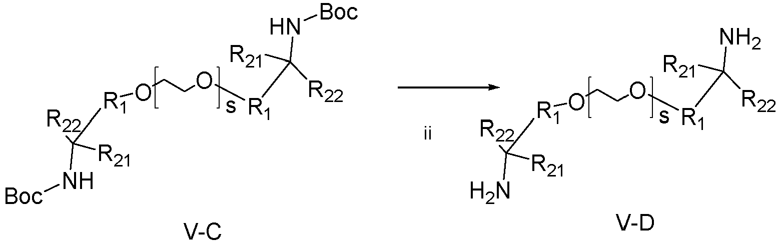

(ii) removing the protective group from the compound of formula V-C to obtain a compound of formula V-D; and

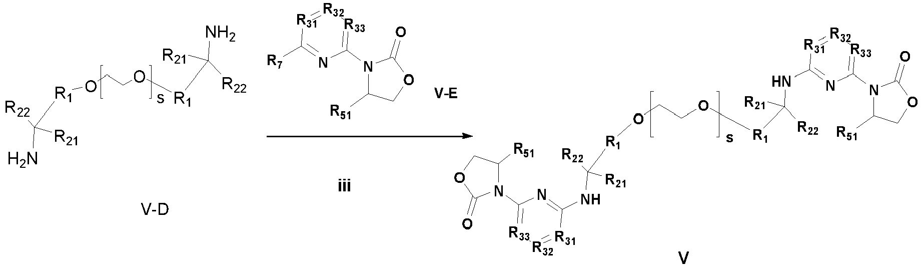

(iii) reacting the compound of formula V-D with a compound of formula V-E to obtain a compound of formula V;

in each formula, R₇ is selected from the group consisting of F, Cl, Br, I, preferably, R₇ is Br or Cl; R₁, R₂₁, R₂₂, R₃₁, R₃₂, R₃₃, R₅₁ and s are as defined above.

In a fifth aspect, the present invention provides use of the compound described in the first aspect for the manufacture of a medicament for (i) inhibiting the activity of mutant IDH, and/or (ii) treating and/or preventing a mutant IDH-mediated disease.

In a sixth aspect, the present invention provides a method for treating and/or preventing a mutant IDH-mediated disease, and the method includes a step for administering a compound or a pharmaceutically acceptable salt thereof described in the first aspect, or a pharmaceutical composition described in the second aspect to a subject in need.

In another preferred embodiment, the subject is a human or non-human mammal; preferably a human.

In another preferred embodiment, the IDH is IDH1.

In another preferred embodiment, the mutant IDH-mediated disease includes cancer.

In another preferred embodiment, the cancer is selected from the group consisting of glioma, glioblastoma, paragangliomas, acute leukemia, prostate cancer, thyroid cancer, colon cancer, chondrosarcoma, bile duct epithelial carcinoma, peripheral T cell leukemia, melanoma, or combination thereof.

It should be understood that within the scope of the present invention, the above-mentioned technical features of the present invention and the technical features specifically described in the following (such as the examples) can be combined with each other to form a new or preferred technical solution, which will not be repeatedly described herein due to space limitation.

### Detailed Description of Embodiments

After an extensive and in-depth research, the inventor unexpectedly found that the inhibitory activity of a multimeric compound formed by small molecule compounds connected by a linking group is greatly improved compared to that of a small molecule compound that does not form a multimer. Experiments show that at the cellular level, the inhibitory effect of the compound of the present invention against the two main mutants has increased by more than a hundredfold. On this basis, the present invention has been completed.

### Definitions

Unless otherwise specified, the term "alkyl" itself or as a part of another substituent refers to a straight or branched chain hydrocarbon having the specified number of carbon atoms (i.e., C1-8 represents 1-8 carbons). Examples of alkyl include methyl, ethyl, n-propyl, isopropyl, n-butyl, tert-butyl, isobutyl, sec-butyl, n-pentyl, n-hexyl, n-heptyl, n-octyl, etc.

Unless otherwise specified, the term "alkenyl" refers to an unsaturated alkyl having one or more double bonds. Similarly, the term "alkynyl" refers to an unsaturated alkyl having one or more triple bonds. Examples of such unsaturated alkyl include ethenyl, 2-propenyl, crotyl, 2-isopentenyl, 2-(butadienyl), 2,4-pentadienyl, 3-(1,4-pentadienyl), ethynyl, 1- and 3-propynyl, 3-butynyl and higher homologues and isomers.

Unless otherwise specified, the term "cycloalkyl" refers to a hydrocarbon ring that has the specified number of ring atoms and is fully saturated or has no more than one double bond between the ring tops. "Cycloalkyl" also refers to bicyclic and polycyclic hydrocarbon rings, such as bicyclo[2.2.1]heptane, bicyclo[2.2.2]octane, etc.

Unless otherwise specified, the term "heterocycloalkyl" refers to a cycloalkyl containing one to five heteroatoms selected from N, O, and S, wherein the nitrogen and sulfur atoms are optionally oxidized, and the nitrogen atom is optionally quaternized. The heterocycloalkyl may be a monocyclic, bicyclic or polycyclic ring system. Non-limiting examples of heterocycloalkyl include pyrrolidine, imidazolidine, pyrazolidine, butyrolactam, valerolactam, imidazolidone, hydantoin, dioxolane, phthalimide, piperidine, 1,4-dioxane, morpholine, thiomorpholine, thiomorpholine-S-oxide, thiomorpholine-S,S-oxide, piperazine, pyran, pyridone, 3-pyrroline, thiopyran, pyrone, tetrahydrofuran, tetrahydrothiophene, quinuclidine, etc. The heterocycloalkyl may be attached to the rest of the molecule though a carbocyclic ring or a heteroatom. For terms such as cycloalkylalkyl and heterocycloalkylalkyl, it refers that the cycloalkyl or heterocycloalkyl is attached to the rest of the molecule through an alkyl or alkylene linker. For example, cyclobutylmethyl-is a cyclobutyl ring attached to the methylene linker of the rest of the molecule.

Unless otherwise specified, the term "alkylene" itself or as a part of another substituent refers to a divalent group derived from an alkane, such as -CH₂CH₂CH₂CH₂-. Alkyl (or alkylene) generally has 1-24 carbon atoms, with those having 10 or fewer carbon atoms being preferred in the invention. "Lower alkyl" or "lower alkylene" is a shorter chain of alkyl or alkylene, usually having 4 or fewer carbon atoms. Similarly, "alkenylene" or "alkynylene" refers to an unsaturated "alkylene" having a double bond or a triple bond, respectively.

Unless otherwise specified, the term "heteroalkyl" itself or in combination with other terms refers to a stable linear or branched or cyclic hydrocarbon or a combination thereof, which consists of the specified number of carbon atoms and 1 to 3 heteroatoms selected from O, N, Si and S, and wherein nitrogen and sulfur atoms are optionally oxidized, nitrogen heteroatoms may be optionally quaternized. The heteroatoms O, N and S may be located at any internal position of the heteroalkyl. The heteroatom Si may be located at any position of the heteroalkyl, including the position where the alkyl group is attached to the rest of the molecule. Examples include -CH₂-CH₂-O-CH₃, -CH₂-CH₂-NH-CH₃, -CH₂-CH₂-N(CH₃)-CH₃, -CH₂-S-CH₂-CH₃, -CH₂-CH₂, -S(O)-CH₃, -CH₂-CH₂-S(O)₂-CH₃, -CH=CHO-CH₃, -Si(CH₃)₃, -CH₂-CH=N-OCH₃ and -CH=CH-N(CH₃)-CH₃. Up to two heteroatoms may be continuous, such as -CH₂-NH-OCH₃ and -CH₂-O-Si(CH₃)₃.

Similarly, unless otherwise specified, the terms "heteroalkenyl" and "heteroalkynyl" themselves or in combination with other terms respectively refer to alkenyl or alkynyl which respectively contains the specified number of carbons and 1 to 3 heteroatoms selected from O, N, Si and S, and wherein nitrogen and sulfur atoms are optionally oxidized, nitrogen heteroatoms may be optionally quaternized. The heteroatoms O, N and S may be located at any internal position of the heteroalkyl.

Unless otherwise specified, the term "heteroalkylene" itself or as a part of another substituent refers to a saturated or unsaturated or polyunsaturated divalent group derived from a heteroalkyl, such as -CH₂-CH₂-S-CH₂CH₂- and -CH₂-S-CH₂-CH₂-NH-CH₂-, -O-CH₂-CH=CH-, -CH₂-CH=C(H)CH₂-O-CH₂- and -S-CH₂-C≡C-. For heteroalkylene, heteroatoms may also occupy any one or two positions at the end of the chain, for example, alkyleneoxy, alkylenedioxy, alkyleneamino, alkylenediamino, etc.

Unless otherwise specified, the terms "alkoxy", "alkylamino" and "alkylthio" or "thioalkoxy" are used in their conventional meanings to refer to those alkyls attached to the rest of the molecule via an oxygen atom, an amino group, or a sulfur atom, respectively. In addition, for dialkylamino, the alkyl can be the same or different, and may also be combined with the nitrogen atom connected to each alkyl to form a 3-7 membered ring. Therefore, a group shown as -NR^{a}R^{b} includes piperidinyl, pyrrolidinyl, morpholinyl, azetidinyl, etc.

Unless otherwise specified, the term "halo" or "halogen" itself or as a part of another substituent refers to a fluorine, chlorine, bromine, or iodine atom. In addition, the term such as "halogenated alkyl" includes monohaloalkyl or polyhaloalkyl. For example, the term "C₁₋₄ halogenated alkyl " includes trifluoromethyl, 2,2,2-trifluoroethyl, 4-chlorobutyl, 3-bromopropyl, etc.

Unless otherwise specified, the term "aryl" refers a polyunsaturated (usually aromatic) hydrocarbon, which can be a monocyclic ring or multiple rings (at most tricyclic ring) fused together or covalently linked. The term "heteroaryl" refers to an aryl group or ring containing 1 to 5 heteroatoms selected from N, O, and S, wherein nitrogen and sulfur atoms are optionally oxidized, and nitrogen atoms are optionally quaternized. Heteroaryl may be attached to the rest of the molecule through heteroatoms. Non-limiting examples of aryl include phenyl, naphthyl and biphenyl, and non-limiting examples of heteroaryl include pyridyl, pyridazinyl, pyrazinyl, pyrimidyl, triazinyl, quinolyl, quinoxalinyl, quinazolinyl, cinnolineyl, phthalazinyl, benzotriazinyl, purinyl, benzimidazolyl, benzopyrazolyl, benzotriazolyl, benzisoxazolyl, isobenzofuryl, isoindolyl, indolizinyl, benzotriazinyl, thienopyridinyl, thienopyrimidinyl, pyrazolopyrimidinyl, imidazopyridine, benzothiazolyl, benzofuranyl, benzothienyl, indolyl, quinolinyl, isoquinolinyl, isothiazolyl, pyrazolyl, indazolyl, pteridyl, imidazolyl, triazolyl, tetrazolyl, oxazolyl, isoxazolyl, thiadiazolyl, pyrrolyl, thiazolyl, furyl, thienyl, etc. The respective substituents of the above aryl and heteroaryl ring systems are selected from the group of acceptable substituents described below.

For brevity, when the term "aryl" is used in combination with other terms, e.g., aryloxy, arylthio, aralkyl, it includes aryl and heteroaryl rings as defined above. Therefore, the term "aralkyl" is meant to include those groups in which the aryl is attached to an alkyl attached to the rest of the molecule, e.g., benzyl, phenethyl, pyridylmethyl, etc.

In some embodiments, the above-mentioned terms, such as "alkyl", "aryl" and "heteroaryl", will include both substituted and unsubstituted forms of the specified groups. The preferred substituents for each type of group are provided below. For brevity, the terms aryl and heteroaryl will refer to the substituted or unsubstituted forms as provided below, while the term "alkyl" and related aliphatic groups refer to the unsubstituted form unless substituted is specified.

The substituents of alkyl (including those groups commonly referred to as alkylene, alkenyl, alkynyl and cycloalkyl) may be various groups selected from the group consisting of -halogen, -OR', -NR'R", -SR', -SiR'R"R‴ -OC(O)R', -C(O)R', -CO₂R',-CONR'R", -OC(O)NR'R", -NR"C(O)R', -NR'-C(O)NR"R‴, -NR"C(O)₂R', -NH-C(NH₂)=NH, -NR'C(NH₂)=NH, -NH-C(NH₂)=NR', -S(O)R', -S(O)₂R',-S(O)₂NR'R", -NR'S(O)₂R", -CN and -NO₂, the number ranges from 0 to (2m'+1), wherein m' is the total number of carbon atoms in such group. R', R" and R‴ each independently represent hydrogen, unsubstituted C₁₋₈ alkyl, unsubstituted heteroalkyl, unsubstituted aryl, aryl substituted by 1-3 halogens, unsubstituted C₁₋₈ alkyl, C₁₋₈ alkoxy or C₁₋₈ thioalkoxy or unsubstituted aryl-C₁₋₄ alkyl. When R' and R" are attached to the same nitrogen atom, they can combine with the nitrogen atom to form a 3-, 4-, 5-, 6- or 7-membered ring. For example, -NR'R" means to include 1-pyrrolidinyl and 4-morpholinyl. The term "acyl", used alone or as part of another group, refers that the two substituents on the carbon closest to the position of attachment of the group are replaced by the substituent =O (such as - C(O)CH₃, -C(O)CH₂CH₂OR', etc.).

As used herein, the term "heteroatom" is intended to include oxygen (O), nitrogen (N), sulfur (S), and silicon (Si).

For the compounds provided herein, the bond from the substituent (usually the R group) to the center of the aromatic ring (e.g., benzene, pyridine, etc.) will be understood to mean the bond that provides connection at any available vertex of the aromatic ring. In some embodiments, the description also includes connections to rings fused to aromatic rings. For example, a bond drawn to the center of the indolebenzene will represent a bond connected to any available vertex of the six- or five-membered ring of indole.

Unless otherwise specified, in the present invention, all appearing compounds are meant to include all possible optical isomers, such as a single chiral compound, or a mixture of various different chiral compounds, i.e., racemates. In all the compounds of the present invention, each chiral carbon atom may optionally be R configuration or S configuration, or a mixture of R configuration and S configuration.

Some compounds of the present invention have asymmetric carbon atoms (optical centers) or double bonds, and racemates, diastereomers, geometric isomers, regioisomers and individual isomers (for example, separated enantiomers) should all be included within the scope of the present invention. When the compounds provided herein have established stereochemistry (represented as R or S, or indicated by a dashed line or wedge-shaped key), a skilled person in the art will understand that those compounds are substantially free of other isomers, for example, at least 80%, 90%, 95%, 98%, 99% and at most 100% are free of other isomers.

In this context, unless otherwise specified, the term "substituted" means that one or more hydrogen atoms in the group are replaced by a substituent selected from the group consisting of halogen, unsubstituted or halogenated C1-C6 alkyl, unsubstituted or halogenated C2-C6 acyl, unsubstituted or halogenated C1-C6 alkyl, -OH, unsubstituted or halogenated, -CN.

As used herein, the term "comprising", "comprise", "including" or "includes" refers that various component can be used together in the mixture or composition of the present invention. Therefore, the terms "mainly consist of" and "consist of" are included in the term "containing".

As used herein, the term "pharmaceutically acceptable" component refers to a substance that is suitable for use in humans and/or animals without excessive adverse side effects, such as, toxicity, irritation, and allergy, that is, a substance with a reasonable benefit/risk ratio.

As used herein, the term "therapeutically effective amount" refers to any amount as described below of a drug, which when used alone or in combination with another therapeutic agent, can promote the regression of the disease, and the regression of the disease is manifested as a decrease in the severity of the disease symptoms, an increase in the frequency and duration of the asymptomatic period of the disease, or prevention of disorder or disability caused by the disease. A "therapeutically effective amount" of the drug of the present invention also includes a "preventively effective amount". The "preventively effective amount" is any amount as described below of a drug, and when the amount of the drug is administered alone or in combination with another therapeutic agent to a subject who is at risk of developing a disease or suffering from recurrence of the disease, the development or recurrence of the disease can be inhibited.

### Compounds of the Invention

The present invention provides compounds for inhibiting mutant IDH. The compounds of the present invention are inhibitors in dimeric or multimeric form.

As used herein, the term "compounds of the present invention" refers to compounds, pharmaceutically acceptable salts, prodrugs, optical isomers, racemates, solvates with the structure of Formula I:

Dₐ-Wₐ-L-W_{b}-D_{b} (I)

in the formula, each group is defined as described in the first aspect.

As used herein, the term "a pharmaceutically acceptable salt" refers to a salt formed by a compound of the present invention and an acid or base suitable for use as a medicine. Pharmaceutically acceptable salts include inorganic salts and organic salts. A preferred class of salts are the salts formed by the compounds of the present invention with acids. Acids suitable for salt formation include, but are not limited to: inorganic acids, such as hydrochloric acid, hydrobromic acid, hydrofluoric acid, sulfuric acid, nitric acid, phosphoric acid, etc.; organic acids, such as formic acid, acetic acid, propionic acid, oxalic acid, malonic acid, succinic acid, fumaric acid, maleic acid, lactic acid, malic acid, tartaric acid, citric acid, picric acid, methanesulfonic acid, benzene methanesulfonic acid, benzenesulfonic acid, etc.; and acidic amino acids, such as aspartic acid, glutamic acid, etc.

In addition to the salt form, the present invention also provides the compound in the form of a prodrug. Prodrugs of the compounds described herein are those compounds that readily undergo chemical changes under physiological conditions to provide the compounds of the invention. In addition, prodrugs can be converted into compounds of the present invention by chemical or biochemical methods in an ex vivo environment. For example, when placed in a transdermal patch reservoir containing a suitable enzyme or chemical reagent, the prodrug can be slowly converted to the compound of the invention. A preferred prodrug is the ester form.

The compound of the present invention may be in an amorphous form, a crystalline form, or a mixture thereof.

Certain compounds of the present invention can exist in unsolvated as well as solvated forms, including hydrated forms. The solvated form is generally equivalent to the unsolvated form and should be included in the scope of the present invention. Certain compounds of the present invention may exist in polymorphic or amorphous forms. Generally, as far as the application considered in the present invention is concerned, all physical forms are equivalent and should be included in the scope of the present invention.

A compound of the present invention may also contain an unnatural proportion of atomic isotopes at one or more of the isotopic atoms constituting the compound. An unnatural proportion of a certain isotope can be defined as from the naturally found amount of the atom concerned to 100% of that atom. For example, radioactive isotopes, such as tritium (³H), iodine-125 (¹²⁵I), or carbon-14 (¹⁴C), or non-radioactive isotopes, such as deuterium (²H) or carbon-13 (¹³C) may be incorporated into the compound. In addition to those uses described in the present application, such isotopic variants may provide additional uses. For example, isotopic variants of the compounds of the invention may have additional uses, including, but not limited to, as diagnostic and/or imaging reagents, or as cytotoxic/radiotoxic therapeutic agents. In addition, isotopic variants of the compounds of the present invention may have altered pharmacokinetic and pharmacodynamic characteristics, thereby helping to increasing safety, tolerability, or efficacy during treatment. Regardless of whether it is radioactive or not, all isotopic variants of the compounds of the present invention should be included within the scope of the present invention.

### Methods of preparation

In the present invention, the active groups such as Da, Db and Dc used to inhibit the mutant IDH protein, or the corresponding active compounds, can be prepared or purchased with reference to the prior art.

Atypical method for preparing the compound of the present invention is provided, and the method comprises:

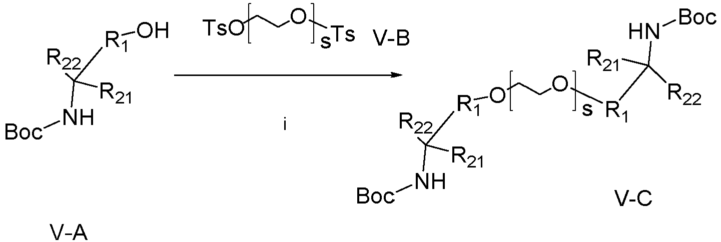

(i) reacting a compound of formula V-A with formula V-B to obtain a compound of formula V-C;

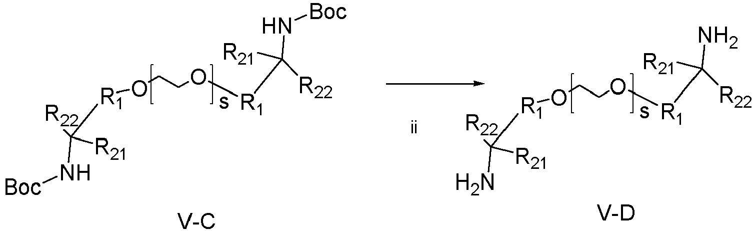

(ii) removing the protective group from the compound of formula V-C to obtain a compound of formula V-D; and

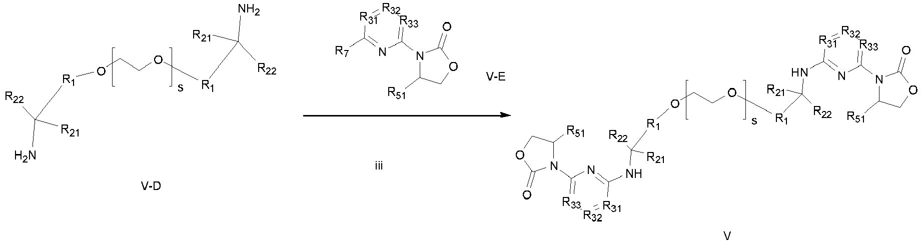

(iii) reacting the compound of formula V-D with a compound of formula V-E to obtain a compound of formula V;

in each formula, R₇ is selected from the group consisting of F, Cl, Br, I, preferably, R₇ is Br or Cl; R₁, R₂₁, R₂₂, R₃₁, R₃₂, R₃₃, R₅₁ and s are as defined above.

Typically, in step (i), usually, the reaction can be carried out in a solvent (such as acetonitrile) under alkaline conditions at room temperature or under heating for 4 hours or more (such as 4-20 hours) to obtain a compound of formula V-C. The compound of formula V-C can be isolated or used directly in the next step.

In step (ii), usually, the reaction can be carried out in a solvent (such as methanol) under acidic conditions at room temperature or under heating for 2 hours or more (such as 2-10 hours) to obtain a compound of formula V.

In step (iii), the reaction can be carried out in a solvent (such as DMSO) under alkaline conditions at room temperature or under heating for 2 hours or more to obtain a compound of formula V.

### Pharmaceutical Compositions and Methods of Administration

The compound of the present invention has excellent inhibitory activity against mutant IDH1 (for example, IDH1/R132H and IDH1/R132C). Therefore, the compound of the present invention and various crystal forms, pharmaceutically acceptable inorganic or organic salts, hydrates or solvates thereof, and pharmaceutical compositions containing the compound of the present invention as the main active ingredient can be used to treat, prevent and alleviate diseases mediated by mutant IDH1.

Preferably, the compounds of the present invention can be used to treat cancer. Representative cancers include, but are not limited to: glioma, glioblastoma, paragangliomas, acute leukemia, prostate cancer, thyroid cancer, colon cancer, chondrosarcoma, bile duct epithelial carcinoma, peripheral T cell leukemia, melanoma, or a combination thereof.

The pharmaceutical composition of the present invention contains a safe and effective amount of the compound of the present invention or a pharmacologically acceptable salt thereof and a pharmacologically acceptable excipient or carrier. The term "a safe and effective amount" means that the amount of the compound is sufficient to significantly improve the condition without causing serious side effects. Generally, the pharmaceutical composition contains 1-2000 mg of the compound of the present invention per dosage, and more preferably, contains 10-500 mg of the compound of the present invention per dosage. Preferably, the term "one dosage " is a capsule or tablet.

"A pharmaceutically acceptable carrier" refers to: one or more compatible solid or liquid fillers or gel substances, which are suitable for human use, and must have sufficient purity and sufficiently low toxicity. "Compatibility" here means that each component of the composition can be blended with the compound of the present invention and with each other without significantly reducing the efficacy of the compound. Examples of pharmaceutically acceptable carriers include cellulose and its derivatives (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerin, mannitol, sorbitol, etc.), emulsifiers (such as Tween^{®}), wetting agents (such as sodium lauryl sulfate), coloring agents, flavoring agents, stabilizers, antioxidants, preservatives, pyrogen-free water, etc.

The method of administration of the compound or pharmaceutical composition of the present invention is not particularly limited, and representative administration methods include, but are not limited to: oral, intratumoral, rectal, parenteral (intravenous, intramuscular, or subcutaneous), and topical administration.

Solid dosage forms for oral administration include capsules, tablets, pills, powders, and granules. In these solid dosage forms, the active compound is mixed with at least one conventional inert excipient or carrier, such as sodium citrate or dicalcium phosphate, or mixed with the following ingredients: (a) fillers or compatibilizers, such as starch, lactose, sucrose, glucose, mannitol, and silicic acid; (b) binders, such as hydroxymethyl cellulose, alginate, gelatin, polyvinylpyrrolidone, sucrose and gum arabic; (c) moisturizers, such as glycerin; (d) disintegrants, such as agar, calcium carbonate, potato starch or tapioca starch, alginic acid, certain complex silicates, and sodium carbonate; (e) sustained-releasing agents, such as paraffin; (f) absorption accelerators, such as quaternary amine compounds; (g) wetting agents, such as cetyl alcohol and glyceryl monostearate; (h) adsorbents, such as kaolin; and (i) lubricants, such as talc, calcium stearate, magnesium stearate, solid polyethylene glycol, sodium lauryl sulfate, or a mixture thereof. In capsules, tablets and pills, the dosage form may also contain buffering agents.

Solid dosage forms such as tablets, sugar pills, capsules, pills, and granules can be prepared with coatings and shell materials, such as enteric coatings and other materials known in the art. They may contain opacifying agents, and the active compound or compound in the composition can be released in a certain part of the digestive tract in a delayed manner. Examples of embedding components that can be used are polymeric substances and waxes. If necessary, the active compound can also be formed into microcapsules with one or more of the above-mentioned excipients.

Liquid dosage forms for oral administration include pharmaceutically acceptable emulsions, solutions, suspensions, syrups, or tinctures. In addition to the active compound, the liquid dosage form may contain inert diluents conventionally used in the art, such as water or other solvents, solubilizers and emulsifiers, such as ethanol, isopropanol, ethyl carbonate, ethyl acetate, propylene glycol, 1,3-butanediol, dimethylformamide and oil, especially cottonseed oil, peanut oil, corn germ oil, olive oil, castor oil and sesame oil, or mixtures of these substances, etc.

In addition to these inert diluents, the composition may also contain adjuvants such as wetting agents, emulsifiers and suspending agents, sweeteners, flavoring agents, and perfumes.

In addition to the active compound, suspensions may contain suspending agents, such as ethoxylated isooctadecanol, polyoxyethylene sorbitol and sorbitan esters, microcrystalline cellulose, aluminum methoxide and agar, or a mixture of these substances, etc.

The composition for parenteral injection may contain physiologically acceptable sterile aqueous or non-aqueous solutions, dispersions, suspensions or emulsions, and sterile powders for reconstitution into sterile injectable solutions or dispersions. Suitable aqueous and non-aqueous carriers, diluents, solvents, or excipients include water, ethanol, polyols, and suitable mixtures thereof.

The dosage form of the compound of the present invention for topical administration includes ointment, powder, patch, spray, and inhalant. The active ingredient is mixed under sterile conditions with a physiologically acceptable carrier and any preservatives, buffers, or propellants that may be required if necessary.

The compounds of the present invention can be administered alone or in combination with other pharmaceutically acceptable compounds.

When using the pharmaceutical composition, a safe and effective amount of the compound of the present invention is administered to the mammal (such as a human) in need of treatment. The administered dose is the effective dosage considered pharmaceutically. For a person with a body weight of 60 kg, the daily dosage is usually 1 to 2000 mg, preferably 20 to 500 mg. Of course, the specific dosage should also consider factors such as the route of administration, the patient's health status, etc., which are within the range of a skilled physician.

The main advantages of the present invention include:
(a) the compound of the present invention is highly effective, non-toxic, and has high inhibitory activity;
(b) the activity of the multimeric compound of the present invention is significantly improved, even hundreds of times, compared to the activity of the small molecule compound that has not been connected;
(c) the compound of the present invention has higher specificity.

The present invention will be further explained below in conjunction with specific embodiments. It should be understood that these embodiments are only used to illustrate the present invention and not to limit the scope of the present invention. The experimental methods without specific conditions in the following examples are usually based on conventional conditions or according to the conditions suggested by the manufacturer. Unless otherwise specified, percentages and parts are percentages and parts by weight.

Description of the compounds involved in the present application

(1) The control compound of the example is AG-120, CAS: 1448347-49-6, commercially available, and the structural formula is as follows:

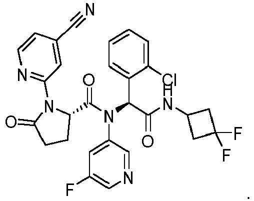

AG-120 (Ivosidenib) is an oral active IDH1 inhibitor with potential anti-tumor activity.

(2) Some of the compounds used in the present invention can be obtained from commercial and/or public compound libraries. For example, some of the compounds of the present invention were purchased from Shanghai Bide Pharmatech Ltd. or Beijing Yinuokai Technology Co., Ltd. or Shanghai Chaolan Chemical Technology Center, or Shanghai Aladdin Biochemical Technology Co., Ltd., or TCI Shanghai Chemical Industry Development Co., Ltd., or Accela ChemBio Co., Ltd., or Shanghai Yunguan Electromechanical Equipment Co., Ltd.

(3) Other compounds of the present invention can be synthesized by a person of ordinary skill using well-known methods in the field of chemical synthesis, such as substitution reactions. For example, the compound of the present invention can be prepared according to the following general scheme.

### Example 1 Synthesis of Compound 1

**(1) Synthesis of (S)-3-(2-chloropyrimidin-4-yl)-4-isopropyloxazolidin-2-one**

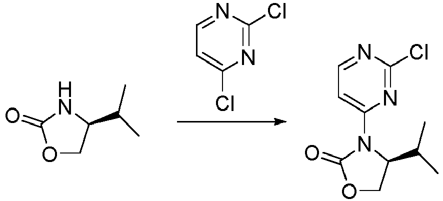

In an ice bath, NaH (60% in mineral oil, 520 mg, 13.0 mmol) was added in batches to a solution of (S)-4-(S)-4-isopropyloxazolidin-2-one (1.50 g, 11.6 mmol) and 2,4-dichloropyrimidine (1.90 g, 12.8 mmol) in DMF. The resulting yellow suspension was stirred overnight at room temperature. Then the mixture was poured into water (150 mL) and extracted with EtOAc (70 mL×3). The combined organic layer was washed with brine (70 ml), dried over MgSO₄ and concentrated. The crude product was purified by column chromatography (petroleum ether: EtOAc = 5:1) to obtain the title compound (2.05 g, yield 73%) as a white solid.

¹H NMR (400 MHz, CDCl₃) δ 8.47 (d, *J* = 6.0 Hz, 1H), 8.18 (d, *J* = 6.0 Hz, 1H), 4.81-4.77 (m, 1H), 4.43-4.34 (m, 2H), 2.64-2.56 (m, 1H), 0.99 (d, *J* = 7.2 Hz, 3H), 0.88 (d, *J* = 7.2 Hz, 3H)

**(2) Synthesis of (ethane-1,2-diylbis(oxy))bis(ethane-2,1-diyl) bis(4-methylbenzenesulfonate)**

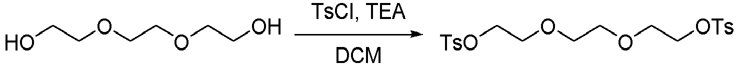

In an ice bath, a solution of 2,2'-(ethane-1,2-diylbis(oxy))diethanol (3.60 g, 23.9 mmol) in DCM was added dropwise to a suspension of TsCl (9.50 g, 49.8 mmol) and TEA (7.27 g, 72.0 mmol) in DCM (70 mL). The mixture was stirred overnight at room temperature. Then the reaction mixture was washed with water (80 mL) and brine (80 mL), dried over MgSO₄ and concentrated. The crude product was purified by silica gel column chromatography (petroleum ether: EtOAc = 5:1) to obtain the desired product (8.1 g, yield 74%) as a white solid.

¹H NMR (400 MHz, CDCl₃) δ7.79 (d,*J* =8.4 Hz, 4H), 7.34 (d,J =8.0 Hz, 4H), 4.15-4.13 (m,4H), 3.67-3.64 (m,4H), 3.53 (s,4H), 2.44 (s, 6H).

**(3) Synthesis of (S,E)-N-((5-bromopyridin-2-yl) ))-2-methylpropane-2-sulfinamide**

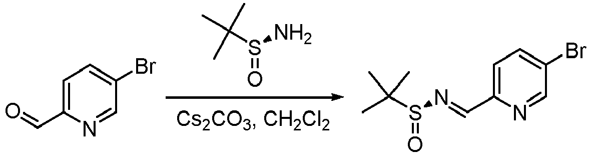

(S)-2-methylpropane-2-sulfinamide (19.5 g, 165 mmol) and CS₂CO₃ (73.5 g, 225 mmol) were added to a solution of 5-bromopicolinaldehyde (27.9 g, 150 mmol) in dichloromethane (200 mL). The resulting mixture was stirred for 5 hours at room temperature. The mixture was then filtered, and the filtrate was concentrated. The residue was purified by column chromatography (dichloromethane) to obtain the desired product (40.0 g, yield 92%) as a white solid.

¹H NMR (400 MHz, CDCl₃) δ8.80 (dd, *J* = 2.0, 0.8 Hz,1H), 8.66 (s,1H), 7.97-7.91 (m, 2H), 1.29 (s, 9H).

**(4) Synthesis of (S)-N-((S)-1-(5-bromopyridin-2-yl)ethyl)-2-methylpropane-2-sulfinamide**

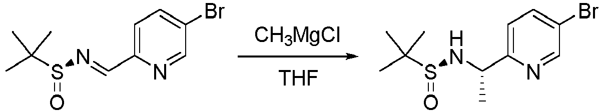

CH₃MgCl (2.5 M in toluene, 75 mL, 187 mmol) was added dropwise to a solution of (S,E)-N-((5-bromopyridin-2-yl)methylene)-2-methylpropane-2-sulfenamide (30.0 g, 104 mmol) in THF (200 mL) at -65°C under N₂ atmosphere. After the addition was complete, the resulting mixture was stirred at -65°Cfor 2 hours. Then NH₄Cl (saturated, 200 mL) was added to the reaction mixture and the mixture was stirred at -65°C for 10 min. The mixture was filtered, and the filtrate was extracted with EtOAc (200 mL×3). The combined organic layer was washed with brine (200 mL), dried over Na₂SO₄, filtered and concentrated to obtain the desired product (31.1 g, yield 100%) as a yellow solid.

¹H NMR (400 MHz, CDCl₃) δ8.60 (d, *J* = 2.4 Hz,1H), 7.78 (dd, *J* = 8.4, 2.4 Hz,1H), 7.21 (d,*J*= 8.4 Hz,1H), 4.57-4.53 (m, 2H), 1.50 (d, *J* = 6.4 Hz, 3H), 1.25 (s, 9H).

**(5) Synthesis of (S)-1-(5-bromopyridin-2-yl)ethanamine**

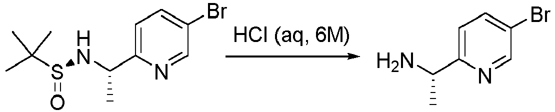

A mixture of (S)-N-((S)-1-(5-bromopyridin-2-yl)ethyl)-2-methylpropane-2-sulfenamide (30.0 g, 98.6 mmol) in HCl (6M aqueous solution, 200 mL) was stirred at room temperature for 2 hours. Then NaOH (2M aqueous solution) was added to adjust pH=7-8, and the aqueous layer was extracted with EtOAc (500 mL×3). The combined organic layer was washed with brine (500 mL), dried over Na₂SO₄ and concentrated to obtain the desired product (19.7 g crude product) as a yellow solid, which was used directly in the next step.

¹H NMR (400 MHz, CDCl₃) δ8.60 (d, *J* = 2.0 Hz,1H), 7.76 (dd, *J* = 8.4, 2.4 Hz,1H), 7.24 (d,*J* = 8.4 Hz,1H), 4.13 (q, *J* = 6.8 Hz,1H), 1.71 (s, 2H), 1.41 (d, *J* = 6.4 Hz, 3H).

**(6) Synthesis of (S)-tert-butyl (1-(5-bromopyridin-2-yl)ethyl)carbamate**

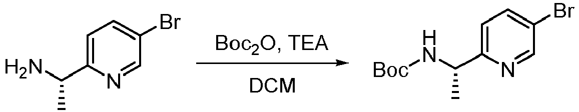

TEA (29.8 g, 296 mmol) and Boc₂O (32.2 g, 148 mmol) were added to a solution of (S)-1-(5-bromopyridin-2-yl)ethylamine (19.7 g, 98.6 mmol) in dichloromethane (300 mL). The resulting mixture was stirred for 2 hours at room temperature. Then the reaction mixture was concentrated, and the crude product was purified by silica gel column chromatography (petroleum ether: EtOAc = 5:1) to obtain the desired product (28.9 g, yield 96%) as a yellow solid. A further 35.2 g product was isolated by chiral preparation. HPLC [chiralpakIG5µm, 20×250 mm; Phase: Hex (hexadecane): EtOH = 85:15; UV = 214nm; F = 18mL / min], the optically isomerized (S)-tert-butyl(1-(5-bromopyridin-2-yl)ethyl)carbamate (27.4 g) was obtained as a white solid.

¹H NMR (400 MHz, CDCl₃) δ8.59 (d, *J* = 2.0 Hz,1H), 7.77-7.76 (m,1H), 7.16 (d,J = 8.4 Hz,1H), 5.50 (br s, 1H), 4.89-4.74 (m,1H), 1.43 (s, 12H).

Chiral chromatography: (condition: chiralpak IB 5 µm, 4.6 × 250 mm; phase: Hex: EtOH = 50: 50; UV = 230 nm; F = 1 mL/min; T = 30°C), Rt = 12.930 min, 100% ee.

**(7) Synthesis of (S)-tert-butyl (1-(5-hydroxypyridin-2-yl)ethyl)carbamate**

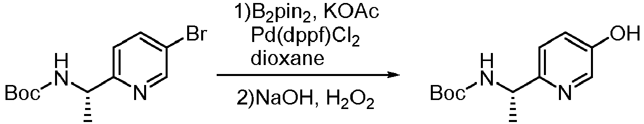

A suspension of (S)-tert-butyl (1-(5-bromopyridin-2-yl)ethyl) carbamate (5.00 g, 16.6 mmol), B₂Pin₂ (5.00 g, 19.9 mmol), KOAc (4.9 g, 49.8 mmol) and Pd(dppf)Cl₂ (364 mg, 0.498 mmol) in dioxane was degassed with N₂. The resulting mixture was stirred overnight at 90°C. The reaction mixture was concentrated and the residue was partitioned between water (150 mL) and EtOAc (100 mL). The aqueous layer was extracted with EtOAc (100 mL×2). The combined organic layer was washed with brine (100 mL), dried over MgSO₄ and concentrated. The crude product was dissolved in THF (100 mL). NaOH (1 M, 41.5 mL, 41.5 mmol) was added and the mixture was stirred for 5 minutes. Then H₂O₂ (30%, 3.76 g, 33.2 mmol) was added dropwise in an ice bath. The resulting mixture was stirred at room temperature for 2 hours. The reaction mixture was poured into aqueous Na₂S₂O₃ (5%, 200 mL) solution. After stirring for 5 minutes, the aqueous layer was extracted with EtOAc (80 mL × 3). The combined organic layer was washed with brine (80 mL×3), dried over MgSO₄ and concentrated. The crude product was purified by a silica gel column (petroleum ether: EtOAc = 2:1) to obtain the desired product (5.77 g crude product) as a brown oil.

¹H NMR (400 MHz, CDCl₃) δ8.12 (s,1H), 7.10 (s,2H), 5.65-5.55 (m,1H), 4.83-4.70 (m, 1H), 1.43-1.40 (m, 12H).

**(8) Synthesis of di-tert-butyl ((1S,1'S)-(5,5'-(((ethane-1,2-diylbis(oxy))bis(ethane-2,1-diyl))bis(oxy))bis(pyridine-5,2-diyl))bis(ethane-1,1-diyl))dicarbamate**

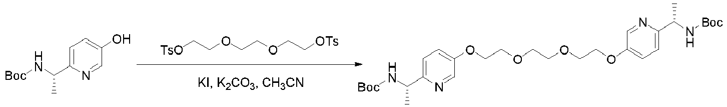

A suspension of (Ethane-1,2-diylbis(oxy))bis(ethane-2,1-diyl)bis(4-methylbenzenesulfonate) (1.05 g, 2.30 mmol), (S)-tert-butyl(1-(5-hydroxypyridin-2-yl)ethyl)carbamate (1.30 g crude product, 5.00 mmol), K₂CO₃ (954 mg, 6.90 mmol) and KI (4 mg, 0.023 mmol) in CH₃CN solution (20 mL) was stirred overnight at 85°C. The reaction mixture was poured into water (100 mL) and extracted with EtOAc (100 mL). The combined organic layer was washed with brine (50 mL), dried over MgSO₄ and concentrated. The crude product was purified by a silica gel column (petroleum ether: EtOAc from 1:1 to 0:1) to obtain the desired product (920 mg, yield 68%) as a yellow oil.

LC-MS [Mobile phase: from 95% water and 5% CH₃CN to 5% water and 95% CH₃CN, within 2.5 min], Rt = 1.56 min; Purity >90% (254 nm); MS calculated value: 590.3; MS measured value: 591.5 [M+H]⁺.

¹H NMR (400 MHz, CDCl₃) δ8.24 (d, *J* = 2.4 Hz,2H), 7.20-7.14 (m,4H), 5.65-5.54 (m,2H), 4.85-4.73 (m, 2H), 4.16 (t, *J* = 4.8 Hz,4H), 3.87 (t, *J* = 4.8 Hz,4H), 3.75 (s, 4H), 1.43-1.41 (m, 24H).

**(9) Synthesis of (1S,1'S)-1,1'-(5,5'-(((ethane-1,2-diylbis(oxy))bis(ethane-2,1-diyl))bis(oxy))bis(pyridine-5,2-diyl))diethanamine tetrahydrochloride**

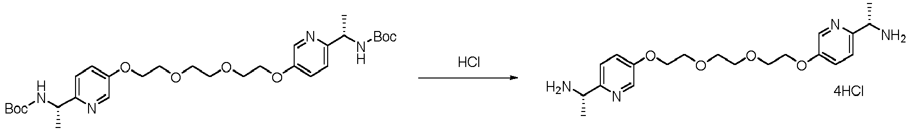

Concentrated hydrochloric acid (2 mL) was added dropwise to a solution of di-tert-butyl ((1S,1'S)-(5,5'-(((ethane-1,2-diylbis(oxy))bis(ethane-2,1-diyl))bis(oxy))bis(pyridine-5,2-diyl))bis(ethane-1,1-diyl))dicarbamate (620 mg, 0105 mmol)) in methanol (10 mL) at room temperature. The resulting mixture was stirred overnight. The reaction mixture was then concentrated to obtain the desired compound (640 mg crude product, yield 100%) as a colorless oil.

LC-MS [Mobile phase: from 95% water and 5% CH₃CN to 5% water and 95% CH₃CN, within 2.5 min], Rt = 0.29 min; MS calculated value: 390.2; MS measured value: 591.3 [M+H]⁺.

**(10) Synthesis of (4S,4'S)-3,3'-(2,2'-(((1S,1'S)-(5,5'-(((ethane-1,2-diylbis(oxy))bis(ethane-2,1-diyl))bis(oxy))bis(pyridine-5,2-diyl))bis(ethane-1,1-diyl))bis(azanediyl))bis(pyrimidine-4,2-diyl))bis(4-isopropyloxazolidin-2-one)**

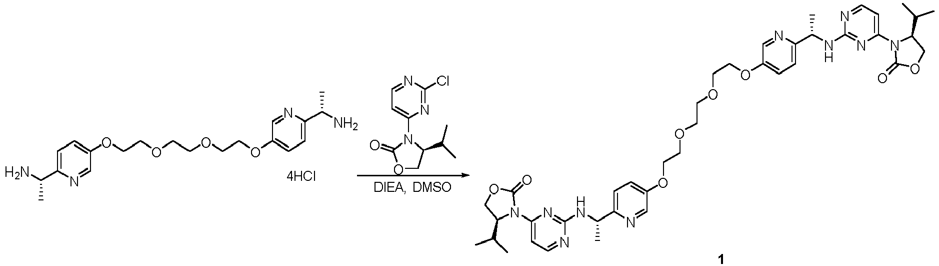

A solution of (1S,1'S)-1,1'-(5,5'-(((ethane-1,2-diylbis(oxy))bis(ethane-2,1-diyl))bis(oxy))bis(pyridine-5,2-diyl))diethanamine tetrahydrochloride (540 mg crude, 0.89 mmol), (S)-3-(2-chloropyrimidin-4-yl)-4-isopropyloxazolidin-2-one (538 mg, 2.23 mmol) and DIEA (1.15 g, 8.9 mmol) in DMSO (8 mL) was stirred overnight at 130°C. After cooling to room temperature, the reaction mixture was poured into water (100 mL) and extracted with EtOAc (50 mL×3). The combined organic layer was washed with water (50 mL), brine (50 mL), dried over MgSO₄ and concentrated. The crude product was purified by a silica gel column (EtOAc: MeOH = 20:1) and further purified by C18 (5-60% CH 3 CN/water) to obtain the desired product 1 (110 mg, yield 15%) as a pale yellow solid.

LC-MS [Mobile phase: from 80% water (0.02% NH₄OAc) and 20% CH₃CN to 20% water (0.02% NH₄OAc) and 80% CH₃CN, within 6.5 minutes], Rt = 4.019 min; Purity: 95.91% (214 nm), 94.56% (254 nm); MS calculated value: 800.4; MS measured value: 801.3 [M+H]⁺.

¹H NMR (400 MHz, CDCl₃) δ8.25 (d, *J* = 2.4 Hz,2H), 8.19 (d, *J* = 6.0 Hz,2H), 7.43 (d, *J=* 6.0 Hz,2H), 7.19-7.14 (m, 4H), 6.03-5.89 (m, 2H), 5.16-4.98 (m, 2H), 4.70-4.60 (m, 2H), 4.28 (t, *J=* 8.4 Hz,2H), 4.22 (dd, *J=* 9.2, 3.2 Hz, 2H), 4.15 (t, *J=* 4.4 Hz,4H), 3.86 (t, *J=* 4.8 Hz,4H), 3.74 (s, 4H), 2.44-1.85 (m, 2H), 1.53 (d, *J=* 6.8 Hz, 6H), 0.78-0.73 (m, 12H).

### Example 2 Synthesis of Compound 2

**(1) Synthesis of ((oxybis(ethane-2,1-diyl))bis(oxy))bis(ethane-2,1-diyl) bis(4-methylbenzenesulfonate)**

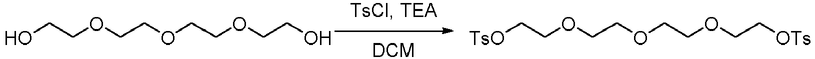

In an ice bath, a solution of 2,2'-((oxybis(ethane-2,1-diyl)bis(oxy))diethanol (3.00 g, 15.4 mmol) in dichloromethane (10 mL) was added dropwise to a suspension of TsCl (6.20 g, 32.3 mmol) and TEA (4.70 g, 46.2 mmol) in DCM (50 mL). The resulting mixture was stirred overnight at room temperature. The reaction mixture was washed with water (60 mL), brine (60 mL), dried over MgSO₄ and concentrated. The crude product was purified by silica gel column chromatography (petroleum ether: EtOAc from 2:1 to 0:1) to obtain the desired product (5.6 g, yield 72%) as a brown oil.

¹H NMR (400 MHz, CDCl₃) δ 7.79 (d,*J* = 8.4 Hz, 4H), 7.34 (d,*J* = 8.0 Hz, 4H), 4.15 (t,J= 4.8 Hz, 4H), 3.68 (t,*J* = 4.8 Hz, 4H), 3.60-3.53 (m, 8H), 2.44 (s, 6H).

**(2) Synthesis of di-tert-butyl ((1S,1'S)-(5,5'-((((oxybis(ethane-2,1-diyl))bis(oxy))bis(ethane-2,1-diyl))bis(oxy))bis(pyridine-5,2-diyl))bis(ethane-1,1-diyl))dicarbamate**

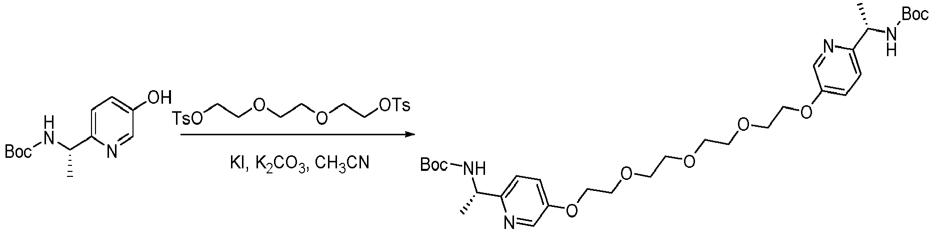

K₂CO₃ (330 mg, 2.39 mmol) and KI (4 mg, 0.024 mmol) were added to a solution of ((oxybis(ethane-2,1-diyl))bis(oxy))bis(ethane-2,1-diyl)bis(4-methylbenzenesulfonate) (400 mg, 0.796 mmol) and (S)-tert-butyl(l-(5-hydroxypyridin-2-yl)ethyl) carbamate (379 mg, 1.59 mmol) in CH₃CN (7 mL). The resulting mixture was stirred overnight at 85°C. The reaction mixture (first batch) was operated with another batch.

K₂CO₃ (659 mg, 4.77 mmol) and KI (8 mg, 0.05 mmol) were added to a solution of ((oxybis(ethane-2,1-diyl))bis(oxy))bis(ethane-2,1-diyl) bis(4-methylbenzenesulfonate) (800 mg, 1.59 mmol) and (S)-tert-butyl(1-(5-hydroxypyridin-2-yl)ethyl) carbamate (796 mg, 3.34 mmol) in CH₃CN (16 mL). The resulting mixture was stirred overnight at 85°C. The reaction mixture combined with the first batch was poured into water (100 mL) and extracted with EtOAc (50 mL×3). The combined organic layer was washed with brine (50 mL), dried over MgSO₄ and concentrated. The crude product was purified by a silica gel column (petroleum ether: EtOAc from 1:1 to 0:1) to obtain the desired product (578 mg, yield 38%) as a pale yellow oil.

¹H NMR (400 MHz, CDCl₃) δ 8.24 (d,*J* = 2.4 Hz, 2H), 7.20-7.14 (m, 4H), 5.59 (br s, 2H), 4.85-4.71 (m, 2H), 4.16-4.14 (m, 4H), 3.87-3.84 (m, 4H), 3.74-3.67 (m, 8H), 1.43-1.41 (m, 24H).

**(3) Synthesis of (1S,1'S)-1,1'-(5,5'-((((oxybis(ethane-2,1-diyl))bis(oxy))bis(ethane-2,1-diyl))bis(oxy))bis(pyridine-5,2-diyl))diethanamine tetrahydrochloride**

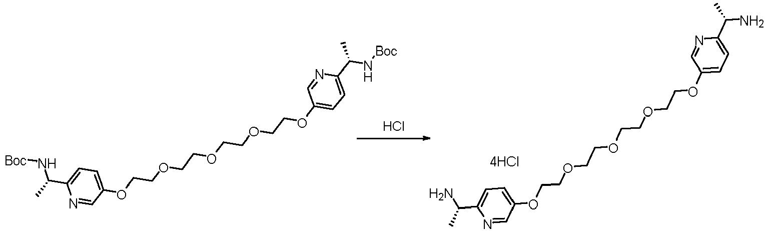

Concentrated HCl (2 mL) was added dropwise to a solution of di-tert-butyl ((1S,1'S)-(5,5'-((((oxybis(ethane-2,1-diyl))bis(oxy))bis(ethane-2,1-diyl))bis(oxy))bis(pyridine-5,2-diyl))bis(ethane-1,1-diyl))dicarbamate (578 mg, 0.911 mmol) in MeOH (10 mL) At room temperature. The resulting mixture was stirred overnight. The reaction mixture was then concentrated to obtain the desired compound (715 mg crude product, yield 100%) as a brown oil.

LC-MS [Mobile phase: from 95% water and 5% CH₃CN to 5% water and 95% CH₃CN, within 2.5 min], Rt = 1.07 min; MS calculated value: 434.3; MS measured value: 435.4 [M+H]⁺.

**(4) Synthesis of (4S,4'S)-3,3'-(2,2'-(((1S,1'S)-(5,5'-((((oxybis(ethane-2,1-diyl))bis(oxy))bis(ethane-2,1-diyl))bis(oxy))bis(pyridine-5,2-diyl))bis(ethane-1,1-diyl))bis(azanediyl))bis(pyrimidine-4,2-diyl))bis(4-isopropyloxazolidin-2-one)**

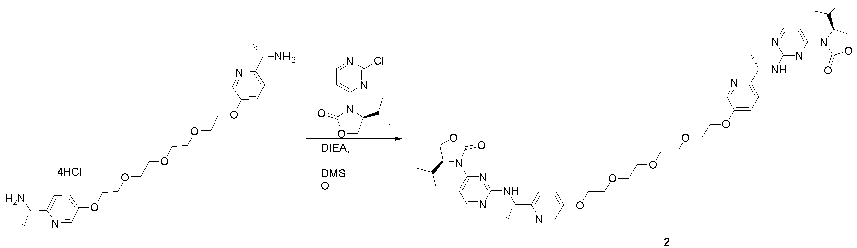

A solution of (1S,1'S)-1,1'-(5,5'-((((oxybis(ethane-2,1-diyl))bis(oxy))bis(ethane-2,1-diyl))bis(oxy))bis(pyridine-5,2-diyl))diethanamine tetrahydrochloride (715 mg crude product, 0.911 mmol), (S)-3-(2-chloropyrimidin-4-yl)-4-isopropyloxazolidin-2-one (660 mg, 2.73 mmol) and DIEA (1.18 g, 9.11 mmol) in DMSO (8 mL) was stirred overnight at 130°C. After cooling to room temperature, the reaction mixture was poured into water (50 mL) and extracted with EtOAc (30 mL × 3). The combined organic layer was washed with water (30 mL), brine (30 mL), dried over MgSO₄ and concentrated. The crude product was purified by silica gel column (EtOAc: MeOH = 25:1) and further purified by C18 (5-60% aqueous CH₃CN solution) to obtain the desired product 2 (155 mg, yield 20%) as a pale yellow solid.

LC-MS [Mobile phase: from 70% water (0.02% NH₄OAc) and 30% CH₃CN to 30% water (0.02% NH4OAc) and 70% CH₃CN, within 6.5 min], Rt = 3.619 min; Purity: 96.91% (214 nm), 96.73% (254 nm); MS calculated value: 844.4; MS measured value: 845.1 [M+H]⁺.

¹H NMR (400 MHz, CDCl₃) δ8.25 (d, *J* = 2.4 Hz,2H), 8.19 (d, *J* = 5.6 Hz,2H), 7.43 (d, *J* = 6.0 Hz,2H), 7.20-7.14 (m, 4H), 6.12-5.90 (m, 2H), 5.16-4.98 (m, 2H), 4.69-4.60 (m, 2H), 4.30-4.21 (m, 4H), 4.15 (t, *J=* 4.8 Hz,4H), 3.85 (t, *J=* 4.8 Hz,4H), 3.73-3.66 (m, 8H), 2.35-1.82 (m, 2H), 1.53 (d, *J=* 6.8 Hz, 6H), 0.78-0.73 (m, 12H).

### Example 3 Inhibitory activity of the compound against mutant IDH1/R132H at the cellular level

2-Hydroxyglutarate dehydrogenase (2HGDH) can reduce NAD+ to NADH in the presence of 2-HG. The later can be quantitatively detected by diaphorase and its substrate Resazurin.

The glioma cell U87MG overexpressing IDH1/R132H mutation was cultured in high-sugar MEM with 1% sodium pyruvate, 10% FBS, and placed in a CO₂ incubator (37°C, 5% CO₂, 95% air).

The cells were digested with trypsin and seeded in a 96-well plate at a density of 1×10⁴ with a medium of 200 µL and cultured in a 37°C incubator overnight. The next day, the test compound was added to a final concentration of 0.1% for DMSO. After culturing for 24 hours, 100 µL of culture medium was vaspirated, and a 10KD Nanosep^{®} ultrafiltration tube (purchased from PALL) was used for centrifugation at 14000 g for 10 minutes. The protein and other components in the culture medium that may interfere with the results were filtered, and the follow-up method was used to detect the content of 2-HG.

### Extracellular 2-HG detecting system:

(1) 50 µL reaction system: reaction buffer (50 mM Tris pH7.5, 100 mM NaCl, 20 mM MgCl₂, 0.05% BSA), in which the final concentration of NAD+ was 40 µM, the final concentration of 2HGDH was 20 nM, and the test sample was added to 5 µL of culture medium; the reaction solution was mixed and centrifuged, and the reaction was carried out for 1 hour at 25°C in the dark;

(2) 25 µL color development system: color development buffer (50 mM Tris pH7.5, 100 mM NaCl, 20 mM MgCl₂, 0.05% BSA), in which the final concentration of diaphorase was 36 µg/mL, and the final concentration of resazurin sodium was 3 µM; 25 µL of the above-mentioned color developing solution was added to the 50 µL reaction system in (1), mixed and centrifuged, and the fluorescence value was measured immediately under Ex544/Em590.

Preparation of standard curve of 2-HG: the stock solution of 2-HG was diluted to 20 µM with reaction buffer, and a 2-fold gradient diluting was performed for a total of 6 points. Afterwards the above-mentioned 2-HG was measured according to the extracellular 2-HG detecting system, and a standard curve was calculated and drawn.

### Calculation of extracellular 2-HG content:

the fluorescence value obtained in the extracellular 2-HG detecting system was calculated using the 2-HG standard curve to calculate the content of 2-HG in the culture medium, and DMSO was used as a negative control to calculate the compound's inhibition agsinst IDH1/R132H mutation producing the 2-HG activity.

The results of the test are shown in Table 1. Wherein, "-" means that the range of inhibitory activity against IDH1/R132H is IC₅₀≥10 µM; "+" means that the range of inhibitory activity against IDH1/R132H is 500 nM≤IC₅₀≤10 µM; "++", "-" mean that the range of inhibitory activity against IDH1/R132H is 5 nM<IC₅₀<500 nM; "+++" means that the range of inhibitory activity against IDH1/R132H is IC₅₀≤5 nM.

**Table 1 Compounds of the present invention and the inhibitory activity against IDH1/R132H**

| Compound number | Structural formula | IC₅₀ (nM) |
|---|---|---|
| 1 | 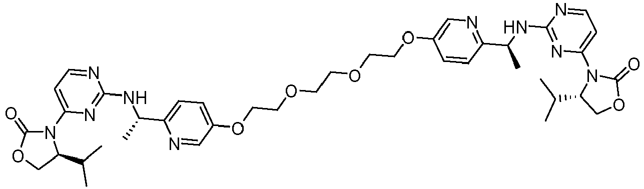 | +++ |
| 2 | 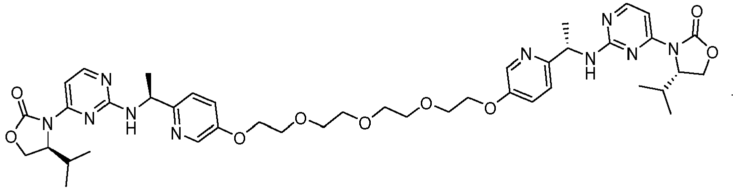 | +++ |
| Ref1 | 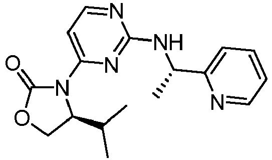 | + |
| IDH125 | 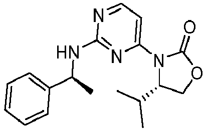 | + |
| AG-120 | 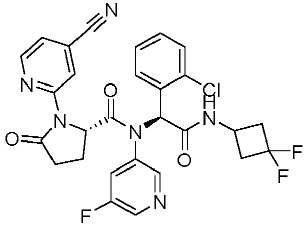 | ++ |

The results showed that compounds 1 and 2 had excellent inhibitory activity against IDH1/R132H (IC₅₀≤5 nM).

### Example 4 The inhibitory activity of the compound against mutant IDH1/R132C at the cellular level

The experimental steps were the same as in Example 3, except that the glioma cell U87MG that overexpresses the IDH1/R132H mutation was replace with the fibrosarcoma cell HT-1080 containing the IDH1/R132C mutation.

The results of the test are shown in Table 2. "-" means that the range of inhibitory activity against IDH1/R132H is IC₅₀≥10 µM; "+" means that the range of inhibitory activity against IDH1/R132H is 500 nM≤IC₅₀≤10 µM; "++", "-" mean that the range of inhibitory activity against IDH1/R132H is 5 nM<IC₅₀<500 nM; "+++" means that the range of inhibitory activity against IDH1/R132H is IC₅₀≤5 nM.

**Table 2 The inhibitory activity of preferred compounds against IDH1/R132C**

| Compound number | IC₅₀(nM) |
|---|---|
| 1 | +++ |
| 2 | +++ |
| Ref1 | + |
| IDH125 | + |
| AG-120 | ++ |

The results showed that compounds 1 and 2 had excellent inhibitory activity against IDH1/R132C (IC₅₀≤5 nM).

### Example 5 The inhibitory activity of the compound against mutant IDH2/R140Q at the cellular level

The experimental steps were the same as in Example 7, except that the glioma cell U87MG overexpressing the IDH1/R132H mutation was replaced with the glioma cell U87MG overexpressing the IDH2/R140Q mutation.

The results of the test are shown in Table 3. Wherein "+" in Table 4 means that the range of inhibitory activity against IDH2/R140Q is IC₅₀≤20 µM; "-" means that the range of inhibitory activity against IDH2/R140Q is IC₅₀>20 µM.

**Table 3 The inhibitory activity of preferred compounds against IDH2/R140Q**

| Compound number | IC₅₀(µM) |
|---|---|
| 1 | - |
| 2 | - |
| Ref1 | - |
| IDH125 | - |
| AG-120 | - |

The results showed that compounds 1 and 2 of the present invention did not inhibit IDH2/R140Q, showing the excellent specificity of the compounds.

All documents mentioned in the present invention are cited as references in the present application, as if each document was individually cited as a reference. In addition, it should be understood that after reading above teaching content of the present invention, a skilled person in the art can make various changes or modifications to the present invention, and these equivalent forms also fall within the scope defined by the appended claims of the present application.

## Claims

1. A compound of formula I,
Dₐ-Wₐ-L-W_{b}-D_{b} (I)
or a pharmaceutically acceptable salt thereof, wherein,
Wa and Wb are each independently absent or selected from the group consisting of O, S, NRa, CO, COO, SO, SO₂, CO-NRₐ, NRₐ-CO, SO-N(Rₐ), N(Ra)-SO, NRₐ-COO, COO-NRₐ, NRₐ-SO₂, SO₂-NRₐ, CS-NRₐ, NRₐ-CS orN(Rₐ)-CO-NRₐ;
wherein Rₐ is each independently selected from the group consisting of H, deuterium, CN, halogen, C1-C6 alkyl, C1-C6 halogenated alkyl, or substituted or unsubstituted C3-C6 cycloalkyl, preferably the substitution is C1-C6 alkyl substitution and/or halogen substitution; preferably, Rₐ is each independently selected from the group consisting of H, deuterium, -CH₃, -C₂H₅, -CH(CH₃)₂, -CH₂CH₂CH₃, cyclopropyl;
L is a linking group shown in formula II
-(X)ₙ (II)
wherein,
n is an integer of 1-50, preferably an integer of 3-40;
each X is the same or different, and each X is independently selected from the group consisting of O, S, substituted or unsubstituted C1-C6 alkylene, substituted or unsubstituted C2-C6 alkenylene, substituted or unsubstituted C2-C6 alkynylene, CO, SO₂, NR_{b}, C(R_{c})₂, substituted or unsubstituted 4- to 10-membered carbocyclic ring, substituted or unsubstituted 4- to 10-membered heterocyclic ring, substituted or unsubstituted 6- to 12-membered aromatic ring, substituted or unsubstituted 5- to 12-membered heteroaromatic ring; or -Wc(T)ₖ-, wherein Wc is a trivalent group, a tetravalent group, or a pentavalent group, and k is 1, 2 or 3; T is -R_{d}-Wa-L'-Wb-D_{c}, wherein Wa and Wb are as described above;
each R_{d} is independently absent or a divalent group selected from the group consisting of substituted or unsubstituted C1-C6 alkylene, substituted or unsubstituted C2-C6 alkenylene, substituted or unsubstituted C1-C6 halogenated alkylene, substituted or unsubstituted C3-C6 cycloalkyl;
L' is absent or a linking group shown in formula II-A:
-(Y)ₘ- (II-A)
wherein,
m is an integer of 1-50;
each Y is the same or different, and each Y is independently selected from the group consisting of O, S, substituted or unsubstituted C1-C6 alkylene, substituted or unsubstituted C2-C6 alkenylene, substituted or unsubstituted C2-C6 alkynylene, CO, SO₂, NR_{b}, C(R_{c})₂, substituted or unsubstituted 4- to 10-membered carbocyclic ring, substituted or unsubstituted 4- to 10-membered heterocyclic ring, substituted or unsubstituted 6- to 12-membered aromatic ring, substituted or unsubstituted 5- to 12-membered heteroaromatic ring;
each R_{b} is independently selected from the group consisting of H, deuterium, C1-C6 alkyl, C1-C6 halogenated alkyl, C1-C6 alkoxy;
each R_{c} is independently selected from the group consisting of H, deuterium, halogen, C1-C6 alkyl, C1-C6 halogenated alkyl, C1-C6 alkoxy, OH, CN;
wherein, the heterocyclic ring or the heteroaromatic ring contains 1, 2 or 3 heteroatoms selected from O, S or N;
with the proviso that, when X is a substituted carbocyclic ring, substituted heterocyclic ring, substituted aromatic ring, or substituted heteroaryl, the substituents on the carbocyclic ring, heterocyclic ring, aromatic ring or heteroaryl may optionally contain 1, 2 or 3 T, wherein T is as defined above;
unless otherwise specified, the term "substituted" means that 1 to 5 hydrogens in the group are each independently replaced by a substituent selected from the group consisting of deuterium, halogen, C1-C6 alkyl, C1-C6 halogenated alkyl, C1-C6 alkoxy, substituted or unsubstituted phenyl, substituted or unsubstituted benzyl, -N(R_{b})₂, -C(R_{c})₃, -CN, -OH,-COORf, -SO₂R_{f}, -NHC(O) R_{f}; wherein R_{f} is each independently selected from the group consisting of hydrogen, deuterium, C1-C6 alkyl, R_{b} and R_{c} are as defined above; and
Dₐ, D_{b} and D_{c} are each independently an active group that inhibits mutant IDH protein.

2. The compound of claim 1, wherein Dₐ, D_{b} and D_{c} are each independently a group shown in formula III: 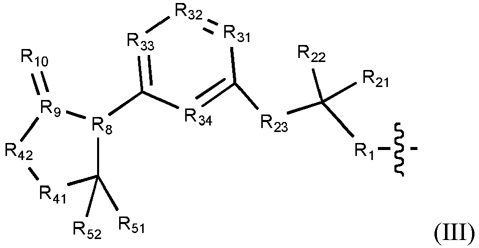 wherein,
R₁ is a divalent linking group;
R₂₁ and R₂₂ are each independently selected from the group consisting of hydrogen, deuterium, halogen, amino, cyano, substituted or unsubstituted C1-C6 alkyl, or R₂₁ and R₂₂ taken together with the carbon atom to which they are attached form a substituted or unsubstituted C3-C5 cycloalkyl; wherein the term "substituted" means that one or more (preferably, 1 to 3) H in the group are replaced by a substituent selected from the group consisting of deuterium, halogen, C1-C6 alkyl;
R₂₃ is selected from the group consisting of NR₆₂;
R₃₁, R₃₂, R₃₃ and R₃₄ are each independently selected from the group consisting of N, CR₆₁;
R₈ is selected from the group consisting of N, CR₆₁;
R₉ is C;
R₁₀ is selected from the group consisting of O, S;
R₄₁ and R₄₂ are each independently selected from the group consisting of O, S, C(R₅₃)₂;
R₅₁ is each independently selected from the group consisting of H, deuterium, substituted or unsubstituted C1-C6 alkyl, substituted or unsubstituted phenyl, substituted or unsubstituted benzyl, substituted or unsubstituted 5- to 7-membered heteroaryl, substituted or unsubstituted diphenylmethyl;
R₅₂ is selected from the group consisting of H, deuterium, C1-C6 alkyl;
or R₅₁ and R₅₂ taken together with the carbon atom to which they are attached form a substituted or unsubstituted C1-C6 (preferably, C1-C4) cycloalkyl, substituted or unsubstituted 5- to 7-membered heterocyclyl;
R₅₃ is each independently selected from the group consisting of H, deuterium, substituted or unsubstituted C1-C6 alkyl, phenyl, benzyl; or two R₅₃ taken together with the carbon atom to which they are attached form a substituted or unsubstituted 3- to 7-membered cycloalkyl or substituted or unsubstituted 4- to 7-membered heterocyclic ring; in the R₅₃ group, the term "substituted" means that 1 to 3 hydrogens in the group are each independently replaced by a substituent selected from the group consisting of deuterium, halogen, hydroxyl, CI-6 alkyl, C1-C6 halogenated alkyl, amino;
R₆₁ is each independently selected from the group consisting of hydrogen, deuterium, halogen (preferably, F, Cl, Br), C1-C6 alkyl, C1-C6 halogenated alkyl; and
R₆₂ is each independently selected from the group consisting of hydrogen, deuterium, C1-C6 alkyl, C1-C6 halogenated alkyl.

3. The compound of claim 1, wherein Dₐ, D_{b} and each D_{c} are each independently a monovalent group selected from the group consisting of:

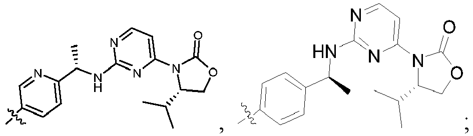

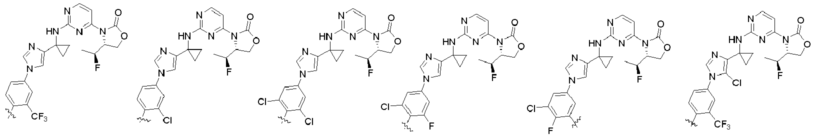

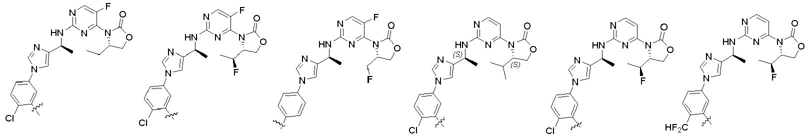

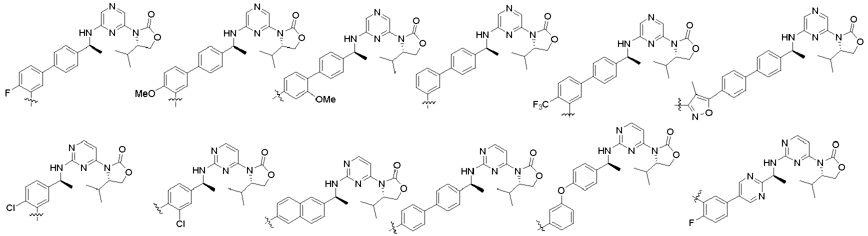

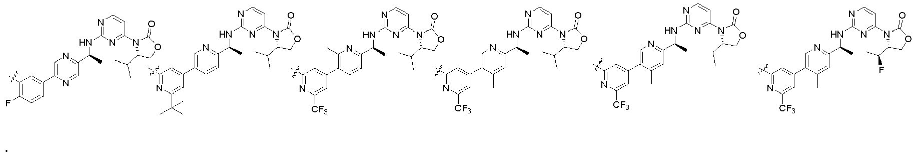

4. The compound of claim 1, wherein L is a linking group shown in formula II-C:
-(Y₁-Y₂-Y₃)_{S}- (II-C)
s is an integer of 1-15;
Y₁, Y₂ and Y₃ are each independently selected from the group consisting of O, S, C1-C6 alkylene, C2-C6 alkenylene, C2-C6 alkynylene, CO, SO₂, NR_{b}, or C(R_{b})₂; or a divalent group formed by substituted or unsubstituted 4- to 10-membered carbocyclic ring, 4- to 10-membered heterocyclic ring, 6- to 12-membered aromatic ring, or 5- to 12-membered aromatic ring losing two hydrogens at any position.

5. The compound of claim 1, wherein the compound is a compound shown in formula IV: 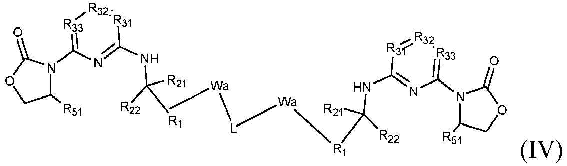 wherein,
R₁ is a divalent linking group;
R₂₁ and R₂₂ are each independently selected from the group consisting of hydrogen, deuterium, halogen, amino, cyano, substituted or unsubstituted C1-C6 alkyl, or R₂₁ and R₂₂ taken together with the carbon atom to which they are attached form a substituted or unsubstituted C3-C5 cycloalkyl; wherein term "substituted" means that one or more H in the group are replaced by a substituent selected from the group consisting of deuterium, halogen, C1-C6 alkyl;
R₃₁, R₃₂ and R₃₃ are each independently selected from the group consisting ofN, CR₆₁; R₅₁ is each independently selected from the group consisting of H, deuterium, substituted or unsubstituted C1-C6 alkyl, substituted or unsubstituted phenyl, substituted or unsubstituted benzyl, substituted or unsubstituted 5- to 7-membered heteroaryl, substituted or unsubstituted diphenylmethyl;
R₆₁ is each independently selected from the group consisting of hydrogen, deuterium, halogen (preferably, F, Cl, Br), C1-C6 alkyl, C1-C6 halogenated alkyl; and
Wa, Wb, and L are as defined in claim 1.

6. The compound of claim 5, wherein the compound is shown in formula V: 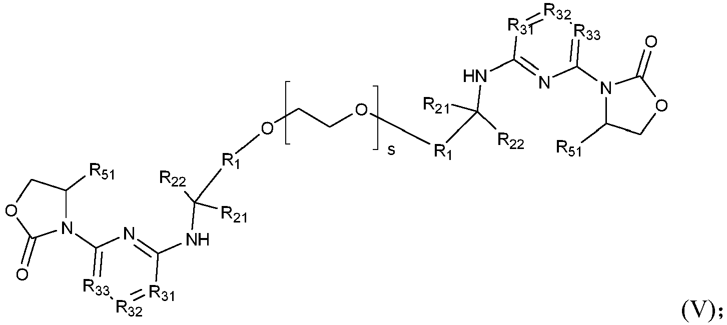 wherein, s is 3, 4, 5, 6 or 7;
R₁, R₂₁, R₂₂, R₃₁, R₃₂ and R₅₁ are as defined in claim 5.

7. The compound of claim 5, wherein the compound is a compound selected from the group consisting of

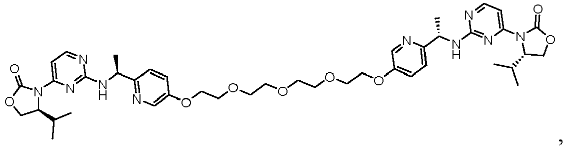

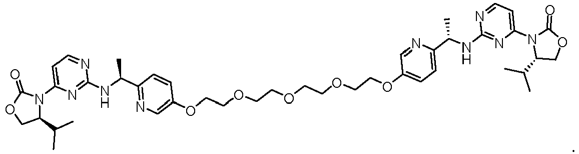

8. A pharmaceutical composition, wherein the composition comprises (i) the compound of claim 1, and (ii) a pharmaceutically acceptable carrier.

9. A method for preparing the compound of claim 6, wherein the method comprises: 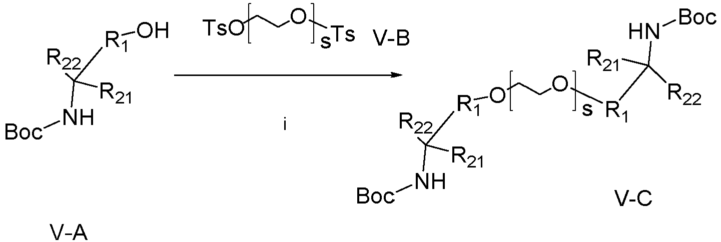
(i) reacting a compound of formula V-A with formula V-B to obtain a compound of formula V-C; 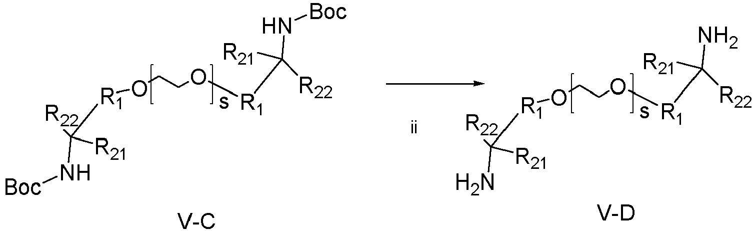
(ii) removing the protective group from the compound of formula V-C to obtain a compound of formula V-D; and 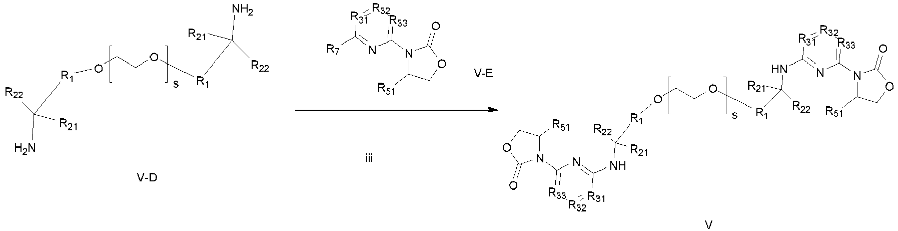
(iii) reacting the compound of formula V-D with a compound of formula V-E to obtain a compound of formula V;
in each formula, R₇ is selected from the group consisting of F, Cl, Br, I, preferably, R₇ is Br or Cl; R₁, R₂₁, R₂₂, R₃₁, R₃₂, R₃₃, R₅₁ and s are as defined in claim 6.

10. Use of the compound of claim 1 for the manufacture of a medicament for (i) inhibiting the activity of mutant IDH, and/or (ii) treating and/or preventing a mutant IDH-mediated disease.

11. The use of claim 10, wherein the mutant IDH-mediated disease includes cancer.

12. The use of claim 11, wherein the cancer is selected from the group consisting of glioma, glioblastoma, paragangliomas, acute leukemia, prostate cancer, thyroid cancer, colon cancer, chondrosarcoma, bile duct epithelial carcinoma, peripheral T cell leukemia, melanoma, or combination thereof.
